Europäisches Patentamt

European Patent Office

Office européen des brevets

(19)

(11) **EP 0 858 510 B1**

(12) **EUROPÄISCHE PATENTSCHRIFT**

(45) Veröffentlichungstag und Bekanntmachung des Hinweises auf die Patenterteilung:
**19.12.2001  Patentblatt 2001/51**

(51) Int Cl.[7]: **C12N 15/54**, C12N 9/10, C12N 1/21, C12P 13/12

(21) Anmeldenummer: **96937217.6**

(22) Anmeldetag: **24.10.1996**

(86) Internationale Anmeldenummer:
**PCT/EP96/04613**

(87) Internationale Veröffentlichungsnummer:
**WO 97/15673 (01.05.1997 Gazette 1997/19)**

(54) **VERFAHREN ZUR HERSTELLUNG VON O-ACETYLSERIN, L-CYSTEIN UND L-CYSTEIN-VERWANDTEN PRODUKTEN**

PROCESS FOR PREPARING O-ACETYLSERINE, L-CYSTEINE AND L-CYSTEINE-RELATED PRODUCTS

PROCEDES DE PREPARATION DE O-ACETYLSERINE, L-CYSTEINE ET PRODUITS APPARENTES A LA L-CYSTEINE

(84) Benannte Vertragsstaaten:
**AT BE CH DE DK ES FI FR GB IT LI NL**

(30) Priorität: **26.10.1995  DE 19539952**

(43) Veröffentlichungstag der Anmeldung:
**19.08.1998  Patentblatt 1998/34**

(73) Patentinhaber: **Consortium für elektrochemische Industrie GmbH
81379 München (DE)**

(72) Erfinder:
 • **LEINFELDER, Walfred
 D-84556 Kastl (DE)**
 • **HEINRICH, Peter
 D-86447 Todtenweis (DE)**

(74) Vertreter: **Potten, Holger et al
Wacker-Chemie GmbH Zentralabteilung Patente, Marken und Lizenzen Hanns-Seidel-Platz 4
81737 München (DE)**

(56) Entgegenhaltungen:
 • **THE JOURNAL OF GENERAL MICROBIOLOGY, Bd. 133, Nr. 3, März 1987, Seiten 515-525, XP000617605 DAGMAR DENK ET AL.: "L-Cysteine biosynthesis in Escherichia coli: Nucleotide sequence and expression of the Serine Acetyltransferase (cysE) gene from the wild-type and a Cysteine -excreting mutant" in der Anmeldung erwähnt**
 • **THE JOURNAL OF GENERAL MICROBIOLOGY, Bd. 133, Nr. 10, Oktober 1987, Seiten 2719-2725, XP000618706 AGNIESZKA E. SIRKO ET AL.: "Identification of the Escherichia coli cysM gene encoding O-Acetylserine sulphydrylase B by cloning with Mini-Mu-lac containing a plasmid replicon" in der Anmeldung erwähnt**

**Beschreibung**

Die vorliegende Erfindung betrifft Verfahren zur Herstellung von O-Acetylserin, L-Cystein und davon abgeleiteten schwefelhaltigen Verbindungen.

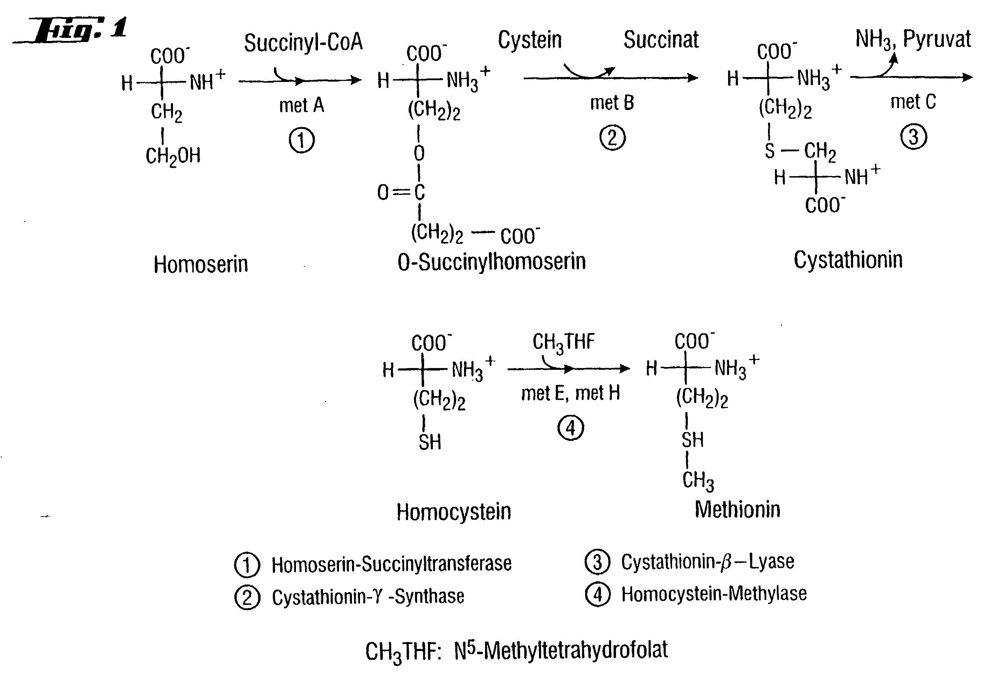

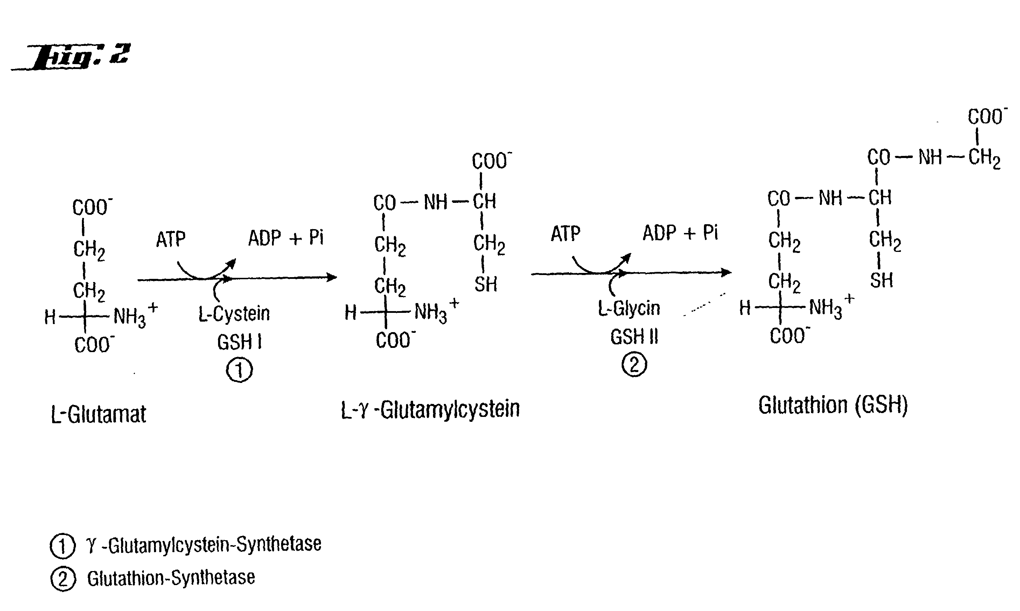

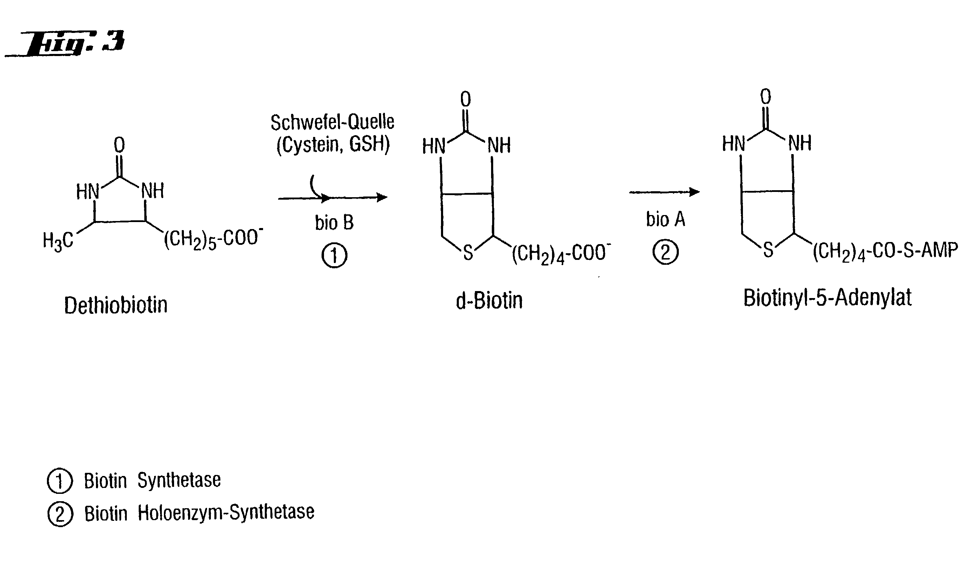

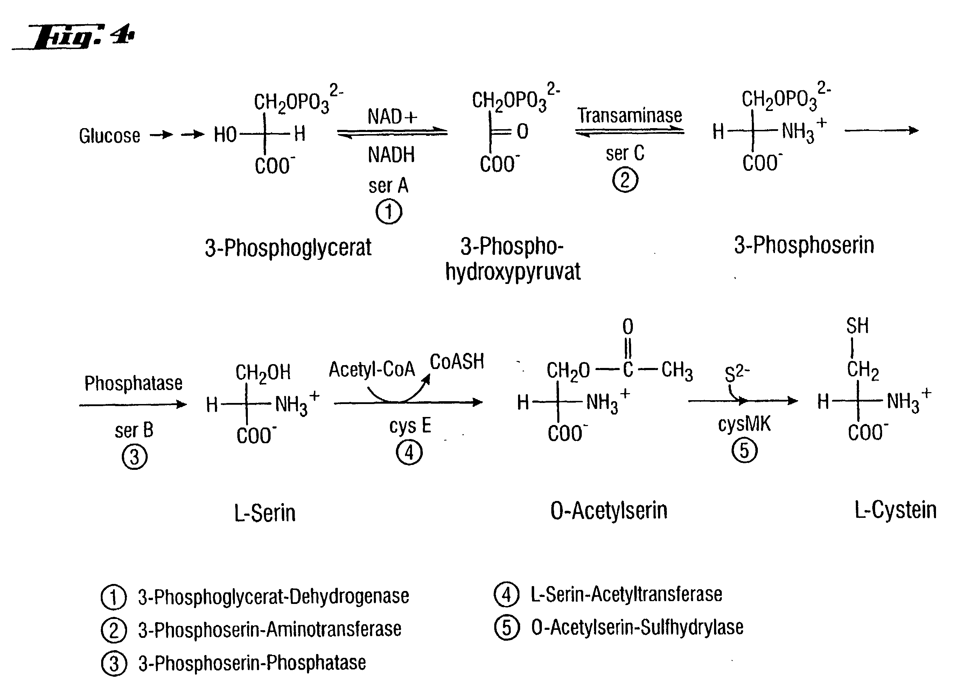

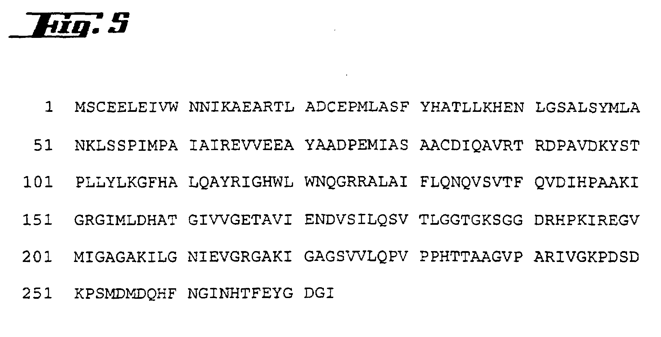

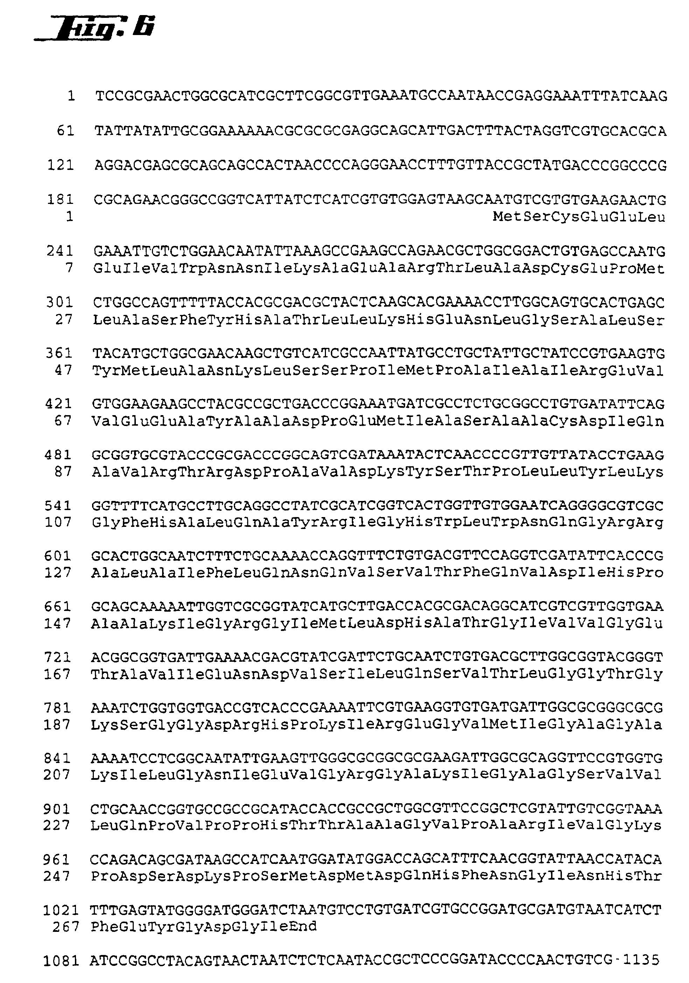

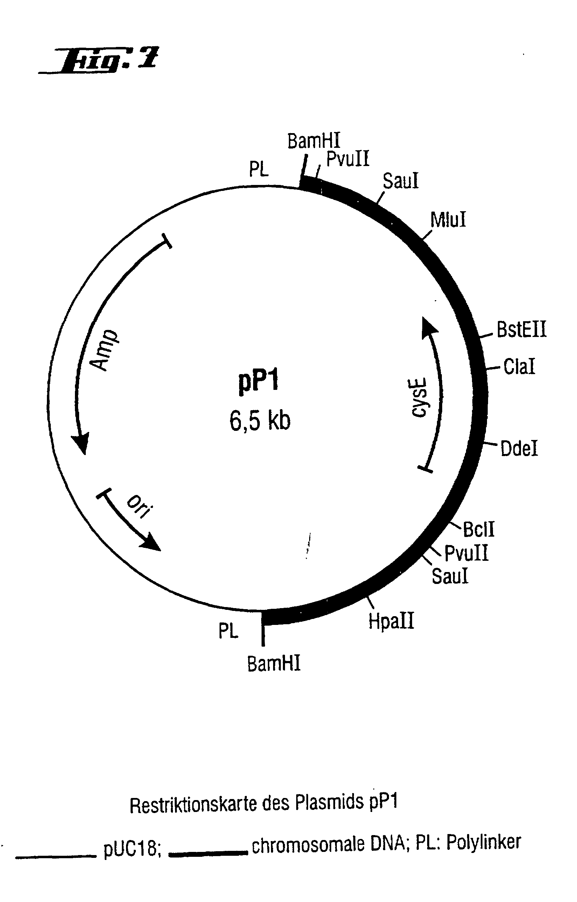

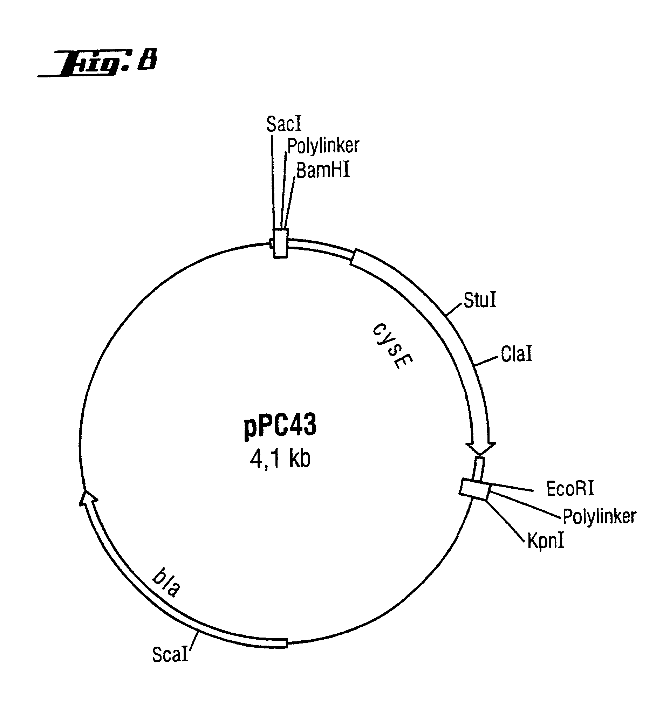

L-Cystein und seine Derivate werden im Pharmabereich (Behandlung von Bronchialkrankheiten), Kosmetiksektor (als Bestandteil in Haarshampoos und Dauerwellenlotionen) und im Lebensmittelbereich (als Antioxidans, Geschmacksverstärker und als Adjuvans bei der Bearbeitung des Teiges) eingesetzt. L-Cystein wird bisher durch Extraktion aus keratinhaltigem Material wie Haaren, Borsten, Hörnern, Hufen und Federn oder durch enzymatische Umsetzung von Vorstufen gewonnen. Eine Überproduktion von L-Cystein durch Mikroorganismen ist sehr wünschenswert, da nicht nur L-Cystein eine wirtschaftlich interessante Verbindung ist, sondern da es zusätzlich, wie aus den Fig. 1-3 hervorgeht, eine wichtige Zwischenverbindung für die Synthese von Glutathion, Methionin und Biotin darstellt.

L-Cystein nimmt in allen Organismen eine Schlüsselposition im Schwefelmetabolismus ein und wird in der Synthese von Proteinen, Glutathion, Biotin, Methionin und anderen, schwefelhaltigen Metaboliten verwendet. Zudem dient L-Cystein als Vorläufer für die Biosynthese von Coenzym A, darüberhinaus kann L-Cystein leicht zu Cystin oxidiert werden. Zwischen der Biosynthese von L-Cystein und anderen Aminosäuren wie L-Serin, Glycin und L-Methionin besteht eine enge Verbindung.

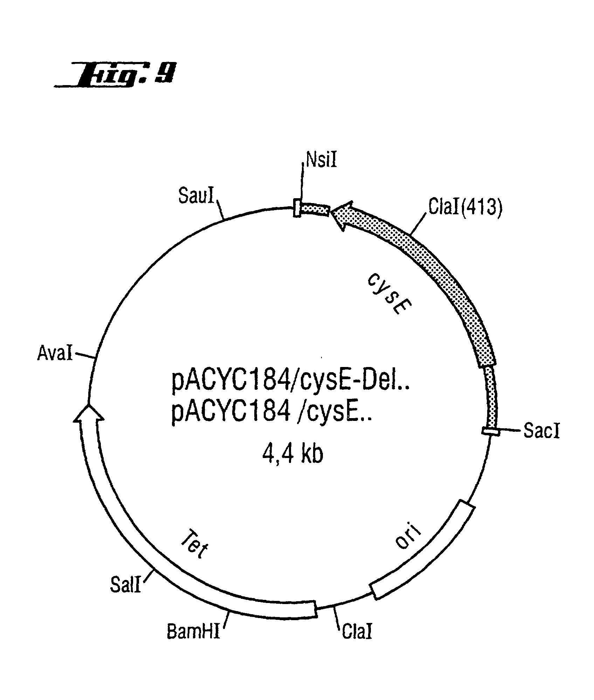

Die Synthese von L-Cystein (Fig. 4) ist in Prokaryonten, insbesondere Bakterien, ausführlich untersucht (Kredich, N. M. and G. M. Tomkins 1966, J. Biol. Chem. **241**: 4955 - 4965; Kredich, N. M., 1987, Biosynthesis of Cysteine. In: Neidhardt F. C., Ingraham, J. L., Magasanik, B., Low. K. B., Schaechter, M., Umbarger, H. E. (eds) Escherichia coli and Salmonella typhimurium: cellular and molecular biology, Vol. 1. American society for Microbiology, Washington D. C., 419 - 428). Die Schlüsselreaktion besteht in der Übertragung einer Acetylgruppe auf das Serin zur Erzeugung von O-Acetylserin 1), gefolgt von dem Austausch der Acetylgruppe gegen die SH-Gruppe, wodurch L-Cystein synthetisiert wird 2).

$$1) \text{ L-Serin + Acetyl-Coenzym A} \rightarrow \text{O-Acetylserin + Coenzym A}$$

$$2) \text{ O-Acetylserin} + H_2S \rightarrow \text{L-Cystein + Acetat}$$

In Mikroorganismen und in Pflanzen dient O-Acetylserin und nicht Serin als unmittelbarer Vorläufer des Kohlenstoffgerüstes für L-Cystein (Kredich, N. M. and G. M. Tomkins 1966, J. Biol. Chem. **241**: 4955 - 4965). Die Reaktion der Acetylgruppenübertragung zur Bildung einer aktivierten Form von L-Serin wird durch die vom cysE-Gen kodierte Serin-Acetyltransferase (EC 2.3.1.30) katalysiert und unterliegt einer strikten Kontrolle durch das Endprodukt L-Cystein. Das Gen für die Serin-Acetyltransferase wurde bereits kloniert und die von der DNS-Sequenz abgeleitete Aminosäuresequenz ist bekannt (Denk, D. and Böck, A. 1987, J. Gen. Microbiol. **133: 515 - 525.**).

Die Bildung von L-Cystein selbst wird katalysiert von zwei O-Acetylserin Sulfhydrylase Isoenzymen (EC 4.2.99.8), kodiert von den Genen cysK (O-Acetylserin-Sulfhydrylase-A) und cysM (O-Acetylserin-Sulfhydrylase-B), eine Reaktion, in welcher O-Acetylserin als β-Alanyl-Donor und $H_2S$ als β-Alanyl-Akzeptor fungiert (Kredich, N. M. and G. M. Tomkins 1966, J. Biol. Chem. **241**: 4955 - 4965), wobei die O-Acetylserin-Sulfhydrylase-A den Hauptanteil an der Cystein-Synthese hat. Zusätzlich ist die O-Acetylserin-Sulfhydrylase-B (cysM) in der Lage, Thiosulfat als Schwefelquelle zu verwerten (Sirko , A. et al., 1987, J. Gen. Microbiol. **133**: 2719-2725). Die O-Acetylserin-Sulfhydrylase-B katalysiert die.Reaktion zwischen O-Acetylserin und Thiosulfat zu S-Sulfocystein, welches dann zu Cystein konvertiert werden kann (Nakamura, T., et al, 1983, J. Bacteriol. 156, 656-662).

Die Endprodukthemmung der Wildtyp-Form der Serin-Acetyltransferase durch L-Cystein ist ein physiologisch bedeutender Faktor in der kinetischen Regulation der Cystein-Biosynthese (Kredich, N. M. 1971, J. Biol. Chem. **246**, 3474 - 3484; Kredich, N. M. and G. M. Tomkins 1966, J. Biol. Chem. **241**, 4955 - 4965). Die Aktivität der Wildtyp-Form der Serin-Acetyltransferase wird durch Cystein gehemmt. Diese Hemmung wurde kinetisch untersucht und zeigte eine kompetitive Charakteristik. Es wurde eine Inhibitorkonstante $K_i = 1,1 \times 10^{-6}$ M in Gegenwart von 0.1 mM Acetyl-Coenzym A und 1 mM L-Serin bestimmt (Kredich, N. M. 1971 and Tomkins G.M. 1966, J. Biol. Chem. 241, 4955 - 4965).

Es ist ein Beispiel aus der Literatur bekannt, daß durch chemische Mutagenese eines Cystein-auxotrophen Stammes mit Methansulfonsäureethylester eine Cystein-prototrophe Revertante isoliert werden kann, deren Serin-Acetyltransferase-Aktivität aufgrund eines Aminosäureaustausches im kodierenden Bereich eine schwach ausgeprägte Endprodukt-Hemmung durch L-Cystein aufweist (Denk, D., Böck, A., 1987, J. Gen. Microbiol. 133: 515 - 525). Die Feedbackresistenz dieser Mutante ist laut der genannten Literaturstelle 10-fach erhöht. Der $K_i$ dieser Mutante liegt somit bei ca. 0,01 mM gegenüber der Wildtypform.

Die vorliegende Erfindung betrifft Serin-Acetyltransferasen die eine im Vergleich zum Wildtyp-Enzym reduzierte Sensitivität gegenüber dem Inhibitor L-Cystein aufweisen und deren Proteinsequenz im Vergleich zur Wildtyp-Sequenz mindestens eine Mutation oder Deletion aufweist, dadurch gekennzeichnet, daß die Mutation im Sequenzbereich von Aminosäure in Position 97 bis einschließlich der Aminosäure in Position 273 liegt oder die Deletion im carboxyterminalen Sequenzbereich ab der Aminosäure in Position 248 bis einschließlich Position 259 liegt, wobei Position 1 das Startmethionin aus Fig. 5 (SEQ ID NO: 1) ist und wobei die Mutation von Met zu Ile in Position 256 ausgeschlossen ist und daß die Serin-Acetyltranferase eine Inhibitorkonstante $K_i$ von 0,02 bis 2,3 mM in Gegenwart von 1mM L-Serin und 0,1 mM Acetyl-CoA hat.

Überraschend wurde gefunden, daß die erfindungsgemäßen Aminosäureaustausche und/oder Aminosäure-Deletionen des Carboxyterminus der Serin-Acetyltransferase zu einer Herabsetzung der Cystein-Sensitivität unter Beibehaltung einer ausreichenden, enzymatischen Aktivität führen.

Erfindungsgemäße Serin-Acetyltransferasen, weisen eine für das Wachstum der sie beinhaltenden Mikroorganismen ausreichende Aktivität auf.

Vorzugsweise umfaßt die Proteinsequenz einer erfindungsgemäßen Serin-Acetyltransferase die Aminosäureaustausche mindestens einer der in Tab. 1a oder 1b genannten cysE Mutanten.

Tab. 1a: Feedbackresistente cysE-Allele mit singulären oder multiplen Aminosäureveränderungen im kodierenden Bereich

Tab. 1a

| cysE-Mutante | Nukleotid-Austausch (Nr.) | Aminosäure-Austausch (Nr.) | $K_i (\mu M)$ | spez. Akt $\mu mol/min \times mg$ |
|---|---|---|---|---|
| cysEII | GGC->AGC (934) | Gly238->Ser238 | 10 | 0,068 |
| cysEIII | GGT->GAT (716) | Gly165->Asp165 | 10 | 0,030 |
| cysEIV | GCT->GTT (932) | Ala237->Val237 | 40 | 0,170 |
| | GGC->AGC (934) | Gly238->Ser238 | | |
| cysEV | GCT->GTT (932) | Ala237->Val237 | | |
| | GGC->AGC (934) | Gly238->Ser238 | 10 | 0,246 |
| | ATG->ATA (990) | Met256->Ile256 | | |
| cysEVI | GGC->AGC (934) | Gly238->Ser238 | 10 | 0,075 |
| | ATG->ATA (990) | Met256->Ile256 | | |

EP 0 858 510 B1

EP 0 858 510 B1

| cysE-Mutante | Nukleotid-Aus-tausch (Nr.) | Aminosäure-Austausch (Nr.) | $K_i(\mu M)$ | spez. Akt $\mu$mol/min x mg |
|---|---|---|---|---|
| cysEVII | GCT->GTT (932) | Ala237->Val237 | 10 | 0,253 |
| cysEVIII | ATG->ATA (990) | Met256->Ile256 | 30 | 0,160 |
|  | GCT->GTT (932) | Ala237->Val237 |  |  |
| cysEX | ACG->GCG (721) | Thr167->Ala167 | 50 | 0,156 |
| cysEXI | ACG->GCG (721) | Thr167->Ala167 | 700 | 0,117 |
|  | GGT->AGT (955) | Gly245->Ser245 |  |  |
| cysEXII | AAA->CAA (511) | Lys97->Gln97 | 40 | 0,254 |
|  | GGC->AGC (934) | Gly238->Ser238 |  |  |
|  | TTT->TTG (1023) | Phe267->Leu267 |  |  |
| cysEXIII | GTT->GCT (713) | Val164->Ala164 | 30 | 0,213 |
|  | TTT->TTG (1023) | Phe267->Leu267 |  |  |

EP 0 858 510 B1

| cysE-Mutante | Nukleotid-Aus-tausch (Nr.) | Aminosäure-Austausch (Nr.) | $K_i$ ($\mu$M) | spez. Akt $\mu$mol/min x mg |
|---|---|---|---|---|
| cysEXIV | ACG->GCG (721) ATG->TAG(988+989) | Thr167->Ala167 Met256->Stop256 | >1000 | 0,453 |
| cysEXVI | GAT->GGT (971) AAG->TAG (973) | Asp250->Gly250 Lys251->Stop251 | 50 | 0,554 |
| cysEXVII | GGT->GAT (716) ACG->GCG (721) | Gly165->Asp165 Thr167->Ala167 | 100 | 0,052 |
| cysEXXIII | ACG->GCG GCT->GTT GGC->AGC | Thr167->Ala167 Ala237->Val237 Gly238->Ser238 | 2300 | 0,085 |

Tab. 1b:

| Feedbackresistente cysE-Allele mit carboxyterminalen Deletionen | | | | |
|---|---|---|---|---|
| **cysE-Mutante** | **Deletierte Aminosäuren** | **Terminale Aminosäure** | **$K_i$ ($\mu$M)** | **spez. Akt. $\mu$mol/min x mg** |
| cysE-Del259 | 14 | His259 | 7.5 | 0,328 |
| cysE-Del258 | 15 | Gln258 | 5 | 0,256 |
| cysE-Del257 | 16 | Asp257 | 7.5 | 0,394 |
| cysE-Del256 | 17 | Met256 | 12.5 | 0,366 |
| cysE-Del255 | 18 | Asp255 | 30 | 0,624 |
| cysE-Del250 | 23 | Asp250 | 20 | 0,405 |
| cysE-Del249 | 24 | Ser249 | 15 | 0,420 |
| cysE-Del248 | 25 | Asp248 | 12.5 | 0,270 |

Erfindungsgemäße Cystein-insensitive Serin-Acetyltransferasen können beispielsweise durch Expression von DNS-Sequenzen, welche für erfindungsgemäße Serin-Acetyltransferasen kodieren, erhalten werden.

Die durch diese DNS-Sequenzen kodierten Enzymvarianten weisen jeweils unterschiedliche Sensitivität gegenüber dem Inhibitor L-Cystein auf, wobei in allen Fällen jedoch zumindest eine im Vergleich zum Wildtyp fünffach erhöhte Resistenz der Serin-Acetyltransferase gegenüber dem Inhibitor L-Cystein gefunden wurde.

Die vorliegende Erfindung betrifft ferner DNS-Sequenzen, welche für erfindungsgemäße Serin-Acetyltransferasen kodieren.

Diese DNS-Sequenzen sind dadurch gekennzeichnet, daß sie im kodierenden DNS Sequenzbereich des jeweiligen cysE-Gens von bp 510 bis bp 1040 mindestens eine Mutation aufweisen, wobei bp 1 die erste Base aus Fig. 6 (SEQ ID NO: 2) ist, wobei die Mutation von Guanin nach Adenin, Thymidin oder Cytosin in Position 990 ausgeschlossen ist.

Im folgenden werden die erfindungsgemäßen DNS-Sequenzen auch als feedbackresistente cysE-Allele bezeichnet.

Diese DNS-Sequenzen lassen sich beispielsweise durch unspezifische oder durch gezielte Mutagenesemethoden aus im folgenden beschriebenen Ausgangsmaterial herstellen.

Unspezifische Mutationen innerhalb der genannten DNS-Region können zum Beispiel durch chemische Agentien (z.B. Nitrosoguanidin, Ethylmethansulfonsäure u.ä.) und/oder durch physikalische Methoden (Miller, J.H., 1972, Experiments in Molecular Genetics, Cold Spring Harbor Laboratory, USA: 113-185) und/oder durch unter bestimmten Bedingungen durchgeführte PCR-Reaktionen erzeugt werden (Gibbs, R.A. 1990, Anal. Chem. 62: 1202-1214).

Methoden zur Einführung von Mutationen an spezifischen Positionen innerhalb eines DNS-Fragments sind bekannt und beispielsweise in folgenden Veröffentlichungen beschrieben: Sarkar, G., Sommer, S.S., 1990, BioTechniques 8: 404-407, beschreiben ortsspezifische Mutagenese mittels PCR; Ausubel, F.M. et al., 1987, S. 8.01-8.3.6 Current Protocols in Molecular Biology, Greene Publishing Associates, beschreiben Methoden zur ortsspezifische Mutagenese mittels M 13 Phagen.

Eine weitere Methode zur Erzeugung feedbackresistenter cysE-Allele besteht in der Kombination verschiedener, zur Feedbackresistenz führender Punktmutationen zu multiplen Mutanten mit neuen Eigenschaften.

Als Ausgangsmaterial für die Mutagenese dient vorzugsweise die DNS des Wildtyp-cysE-Gens oder ein durch Mutation inaktiviertes cysE-Gen oder ein mutiertes und bereits für eine feedbackresistente Serinacetyltransferase kodierendes cysE-Gen.

Das zu mutierende cysE-Gen kann chromosomal oder extrachromosomal kodiert sein.

Das, beispielsweise das Wildtyp-cysE-Gen umfassende, Ausgangs-DNS-Fragment wird mit bekannten Standardtechniken zur Herstellung rekombinanter DNS auf einen Vektor rekombiniert. Durch Anwendung der vorgenannten MutageneseMethoden werden ein oder mehrere Nukleotide der DNS-Sequenz so verändert, daß die nun durch das Gen kodierte Aminosäuresequenz mindestens eine Mutation im Sequenzbereich von Position 97 bis einschließlich der Aminosäure in Position 273 aufweist oder mindestens eine Deletion im carboxyterminalen Sequenzbereich ab der Aminosäure in Position 227 vorhanden ist, wobei Position 1 das Startmethionin aus Fig. 5 (SEQ ID NO: 1) ist und wobei die Mutation von Met zu Ile in Position 256 ausgeschlossen ist.

Mit den beschriebenen Techniken lassen sich in ein beliebiges cysE-Gen eine oder mehrere Mutationen im genannten DNS-Bereich einführen, die bewirken, daß die kodierte Serin-Acetyltransferase eine zur Cystein-Insensitivität führende Aminosäuresequenz besitzt.

Im Anschluß an die beispielsweise wie beschrieben durchgeführte Mutagenese erfolgt die Selektion der Mutanten mit dem gewünschten Phänotyp beispielsweise durch Plattierung auf Cystein-freies Medium und anschließender Bestimmung des Ausmaßes der Cystein-Sensitivität der mutierten Serin-Acetyltransferase.

Ein weiterer Gegenstand der Erfindung sind Mikroorganismen, welche die feedbackresistente cysE-Allele enthalten.

Solche Stämme von Mikroorganismen sind dadurch gekennzeichnet, daß sie einen zumindest durch ein feedbackresistentes cysE-Allel deregulierten Cysteinstoffwechsel besitzen.

Da bei allen Mikroorganismen der Cysteinstoffwechsel prinzipiell über denselben, an sich bekannten Stoffwechselweg verläuft und die zur Herstellung der erfindungsgemäßen Stämme anzuwendenden Techniken z.B. aus Standardlehrbüchern allgemein bekannt und auf alle Mikroorganismen anwendbar sind, sind erfindungsgemäße Stämme aus beliebigen Mikroorganismen herstellbar.

Bevorzugt geeignet zur Herstellung eines erfindungsgemäßen Stammes sind Bakterien.

Besonders bevorzugt geeignet sind gram-negative Bakterien, insbesondere E. coli.

Ein weiterer Gegenstand der Erfindung ist die Herstellung von L-Cystein oder von L-Cystein abgeleiteten Produkten durch Kultivierung erfindungsgemäßer Mikroorganismen.

Die feedbackresistenten cys-E Allele ermöglichen eine Aufhebung der Kontrolle eines wichtigen, biosynthetischen Kontrollpunktes, wodurch die Produktion zahlreicher, stromabwärts von diesem Kontrollpunkt liegenden Verbindungen verstärkt wird. Dazu zählen insbesondere O-Acetylserin, L-Cystein und L-Cystein-verwandte Produkte. L-Cystein-verwandte Produkte sind alle von L-Cystein abgeleiteten Produkte, d.h. schwefelhaltige Verbindungen, zu deren Herstellung L-Cystein erforderlich ist. Beispiele für solche Produkte sind 2(R,S)-Methyl-Thiazolidin-2(R,S),4(R)-dicarbonsäure, Homocystein, Methionin, Biotin und Glutathion.

Die feedbackresistenten cys-E Allele werden zur Expression des veränderten Serin-Acetyltransferase-Enzyms mittels üblicher Verfahren in einen Wirtsstamm transformiert. Das "Screening" nach Stämmen mit veränderten Serin-Acetyltransferase-Eigenschaften erfolgt beispielsweise mittels der im folgenden beschriebenen Methoden.

Zur Bestimmung des Ausmaßes der Cystein-Insensitivität des veränderten Enzyms wird zunächst in einem semiquantitativen, sog. Kreuzfütterungstest die Cysteinausscheidung der Stämme gemessen. Dazu werden die zu testenden Stämme auf Cystein-freies Minimalmedium, dem ein Cystein-auxotropher Indikatorstamm zugesetzt ist, ausgebracht. Die Wachstumszone des Indikatorstammes um den jeweiligen Impfstrich (Halo) dient als semiquantitatives Maß für die Cysteinausscheidung. Alle Stämme die im Kreuzfütterungstest ein Halo mit einem Radius > 2 mm aufweisen, werden als "positiv im Kreuzfütterungstest" bezeichnet. Mit den selektierten Stämmen wird zur Bestimmung des Ausmaßes der Cysteintoleranz der veränderten Serin-Acetyltransferase ein Enzymaktivitätstest durchgeführt.

Für die Bestimmung der Cystein-Sensitivität der Serin-Acetyltransferase kann jede Methode benützt werden, die es erlaubt, die Aktivität dieses Enzyms in Anwesenheit von Cystein zu bestimmen. Beispielsweise kann die Bestimmung der Serin-Acetyltransferase-Aktivität nach der von Kredich und Tomkins beschriebenen Methode, J. Biol. Chem. 241: 4955 - 4965 (1966), vorgenommen werden. Bei diesem Test enthält das Enzym-Testgemisch das Substrat L-Serin und den Cofaktor Acetyl-Coenzym A. Die Reaktion wird durch Enzymzugabe gestartet und über die Abnahme der Absorption bei 232 nm, die durch Spaltung der Thioesterbindung im Acetyl-Coenzym A hervorgerufen wird, in einem Spektralphotometer verfolgt.

Der beschriebene Enzymtest eignet sich für die Bestimmung der Cystein-Sensitivität jedes Serin-Acetyltransferase-Enzyms, einschließlich der Enzyme mit modifiziertem Carboxy-Terminus. Die Hemmung der Serin-Acetyltransferase-Aktivität wird in Anwesenheit verschiedener Konzentrationen von L-Cystein im Reaktionsansatz getestet. Die katalytische Aktivität der verschiedenen Serin-Acetyltransferase-Enzyme wird in An- und Abwesenheit von L-Cystein bestimmt und daraus die Hemmkonstante $K_i$ ermittelt (Kredich und Tomkins, J. Biol. Chem., 241, 4955-4965 (1966)).

In den meisten Fällen bevorzugt wird eine enzymatisch aktive Serin-Acetyltransferase mit einer verringerten Cystein-Sensitivität. Für andere Vorhaben kann eine gleichzeitige Reduzierung der Endprodukt-Sensitivität und der katalytischen Aktivität erstrebenswert sein.

In der Regel ist eine starke Überexpression einer Endprodukt-resistenten Serin-Acetyltransferase nicht wünschenswert, da das dabei zu viel gebildete O-Acetylserin, L-Cystein oder davon abgeleitete Metaboliten sich in der Zelle anhäufen, diese toxifizieren und eine Selektion von Mutanten mit reduzierter Serin-Acetyltransferase-Aktivität hervorrufen könnte. Deshalb werden die feedbackresistenten cysE-Allele bevorzugterweise als singuläre Kopien mittels üblicher Verfahren in das Genom integriert.

Methoden für die Integration singulärer Gene in das Chromosom mit Hilfe geeigneter Vektoren sind Stand der Technik (z.B. Winans et al., 1985; J .Bacteriol. 161: 1219 - 1221; Shevell et al., 1988; J. Bacteriol. 170: 3294 - 3296; Kulakauskas et al. 1991, J. Bacteriol. 173: 2633 - 2638).

Es ist ebenfalls bevorzugt, die feedbackresistenten Serin-Acetyltransferasen auf Plasmiden mit niedriger Kopienzahl zu exprimieren.

Bekanntermaßen weist der Expressionsvektor neben dem feedbackresistenten cys-E Allel vorzugsweise noch die im folgenden beschriebene, zusätzlichen Elemente auf.

Die auf dem Vektor befindlichen, kodierenden Sequenzen sind vorteilhafterweise mit regulatorischen Elementen, die für die Expression der kodierenden Sequenzen in dem gewünschten Ausmaß notwendig sind, verknüpft.

Beispiele dieser regulatorischen Elemente sind Promotoren, ribosomale Bindungsstellen und Terminations-Sequenzen. In den meisten Fällen werden die nativen, regulatorischen cysE-Sequenzen für die Expression der erfindungsgemäßen Mutanten verwendet. Es können jedoch auch beliebige andere regulatorische Sequenzen eingesetzt werden.

Neben den regulatorischen Elementen befinden sich auf dem Expressionsvektor vorzugsweise auch Sequenzen, die für selektive Marker und/oder Reporter-Gene kodieren. Die Expression derartiger Selektionsmarker erleichtert die Identifizierung von Transformanten. Als Selektionsmarker geeignet sind Gene, die für eine Resistenz gegenüber z.B. Ampicillin, Tetracyclin, Kanamycin, Chloramphenicol oder anderen Antibiotika kodieren.

Wenn die erfindungsgemäße Mutante extrachromosomal repliziert werden soll, sollte der Plasmidvektor vorzugsweise einen Ursprungspunkt der Replikation enthalten. Die Strategien zur Integration von Genen in das Chromosom mit Hilfe von Vektoren, denen der Ursprungspunkt der Replikation entfernt wurde, sind Stand der Technik (Winans et al., 1985; J. Bacteriol. 161: 1219 - 1221; Shevell et al., 1988; J. Bacteriol. 170: 3294 - 3296; Kulakauskas et al. 1991, J. Bacteriol. 173: 2633 - 2638).

Beispiele für Vektoren, die in E. coli autonom replizierbar sind, sind bei Pouwels, P.H., Enger-Valk, B.E., Brammer, W.J. 1985, Cloning Vectors, Elsevier, Amsterdam aufgeführt.

Solche Vektoren sind beispielsweise:

- Plasmide mit hoher Kopienzahl wie z.B. pBR322, pUC18
- Plasmide mit mittlerer bis niedriger Kopienzahl wie z.B. pACYC184, pACYC177 und pSC101
- Phagenvektoren wie z.B. M13-Vektoren.

Bevorzugt geeignet sind Vektoren mit mittlerer bis niedriger Kopienzahl; besonders bevorzugt sind Vektoren mit einem p15A-Replikon, wie pACYC184 (ATCC37033) oder pACYC177 (ATCC37031) geeignet.

Eine große Anzahl von Vektoren für andere Bakterien sind ebenfalls in der Literatur beschrieben (Pouwels, P.H., Enger-Valk, B.E., Brammer,W.J. 1985, Cloning Vectors, Elsevier, Amsterdam). Mit diesen Vektoren ist die Expression der erfinungsgemäßen Mutanten in anderen Bakterien möglich.

Geeignete rekombinante Vektoren können mit den Standardtechniken zur Herstellung rekombinanter DNS erzeugt werden. Diese Techniken sind ausführlich in Standardlehrbüchern dargestellt.

Ein geeigneter Wirtsstamm wird mit einem Expressionsvektor, der die für eine Cystein-insensitive Serin-Acetyltransferase kodierende Transkriptionseinheit enthält, transformiert.

Als Wirtsstämme werden Stämme, die Cystein-sensitive Proteine enthalten, wie z.B. Prokaryonten oder Hefen verwendet.

Vorzugsweise werden E.coli Wildtypstämme oder Stämme verwendet, in welchen das endogene cysE-Gen inaktiviert ist und durch ein erfindungsgemäße cysE-Gen komplementiert wird. Derartige Zellsysteme eignen sich für die Überproduktion von L-Cystein und daraus abgeleiteten Metabolite.

Bei der Fermentation von Stämmen, enthaltend mindestens ein feedbackresistentes cysE-Allel, zeigt sich, daß Stämme, die ein feedbackresistentes cysE-Allel mit einem $K_i$-Wert zwischen 0,015 und 2,3 mM in Gegenwart von 1 mM L-Serin und 0,1 mM Acetyl-CoA beherbergen, signifikant gößere Mengen an Cystein ausscheiden.

Erfindungsgemäße Serin-Acetyltransferasen lassen sich auch durch Verwendung von Antisense-RNA erzeugen. Es gehört zum Stand der Technik, durch die sogenannte Umkehrgenetik (Reverse Genetik) unter Verwendung von Antisense-RNA gezielt Genaktivität zu blockieren oder zu modifizieren (Inouye, 1988, Gene 72: 25 - 34). Antisense-RNA ist das Transkriptionsprodukt desjenigen DNS-Stranges, der komplementär zu dem für das Protein kodierenden Strang ist. Es ist möglich, die Cystein-Sensitivität der Serin-Acetyltransferase in vivo dadurch zu reduzieren, daß man über Expressionsvektoren Antisense-RNAs produziert, die zu einem definierten Bereich des 3'-kodierenden Stranges der nativen oder transformierten cysE-Gene komplementär sind. Hierbei lagert sich die Antisense-RNA spezifisch an Zielsequenzen der cysE-mRNA an und verursacht dadurch die Synthese von erfindungsgemäßen Serin-Acetyltransferase-Enzymen, die am Carboxyterminus verkürzt sind und analog den Deletionsmutanten von Beispiel 3 gegenüber dem Inhibitor L-Cystein eine verringerte Sensitivität aufweisen.

Eine zusätzliche Aufgabe war es, für eine optimale Cysteinüberproduktion mittels erfindungsgemäßer Mikroorganismen geeignete Schwefelquellen bereitzustellen.

Überraschenderweise wurde gefunden, daß eine intrazelluläre Überproduktion von O-Acetylserin, ausgelöst durch Verwendung der erfindungsgemäßen Serin-Acetyltransferase-Mutanten in Verbindung mit einem geeigneten Nährmedium, zu einem deutlichen Anstieg der extrazellulären Cysteinkonzentration führt. Demnach sind die erfindungsgemäßen Serin-Acetyltransferase-Mutanten geeignet für eine Cystein-Überproduktion.

Hierfür muß einem erfindungsgemäße Mikroorganismus im Produktionsmedium eine ausreichende Menge an Schwefeldonoren bereitgestellt werden.

Geeignete Schwefeldonoren für eine Cysteinüberproduktion sind alle anorganischen Schwefelverbindungen. Bevorzugt sind Sulfate, Sulfite, Sulfide, Dithionite, und Thiosulfate geeignet. Besonders bevorzugt ist Thiosulfat zur optimalen Cysteinproduktion geeignet.

Eine weitere Steigerung der Cysteinausbeute kann durch zusätzliche Überexpression der sulfatreduzierenden (kodiert durch die Gene cysD, C, H, G, I,J) und der sulfhydrierenden Enzyme (kodiert von den Genen cysK und cysM) erzielt werden.

Eine weitere Steigerung der Cysteinausbeute ist möglich

a) durch eine Deregulierung des Regulatorproteins cysB auf Genebene im Sinne einer konstitutiven Expression. Das cysB-Protein fungiert als übergeordnetes Regulationsprotein der Cystein-Biosynthese in E.coli (Kredich, N. M., 1987, Biosynthesis of Cysteine. In: Neidhardt F. C., Ingraham, J. L., Magasanik, B., Low. K. B., Schaechter, M., Umbarger, H. E. (eds) Escherichia coli and Salmonella typhimurium: cellular and molecular biology, Vol. 1. American society for Microbiology, Washington D. C., 419 - 428).
b) durch Kombination von ser-A Genen, ausgewählt aus der Gruppe serA-Wildtyp und serA-Genen kodierend für eine Phosphoglyceratdehydrogenase mit verringerter Serinsensitivität mit erfindungsgemäßen cys-E Genen.
c) durch externe Zufütterung von Serin.

Bevorzugterweise wird das Gen der nativen, Cysteinsensitiven Serin-Acetyltransferase im Wirtsstamm inaktiviert, so daß eine alleinige Synthese der über Transformation in den jeweiligen Stamm eingeführten Cystein-insensitiven Serin-Acetyltransferase gewährleistet ist. Es existieren zahlreiche Vorschriften über die Inaktivierung nativer Gene in E. coli (Hamilton et al., 1989, J. Bacteriol. 171: 4617 - 4622; Russel et al., 1989, J. Bacteriol. 171: 2609 - 2613; Shen and Huang, 1986, Genetics **112**: 441 - 457; Jasin and Schimmel, 1984, J. Bacteriol. 159: 783 - 786).

Ebenso bevorzugt wird die Integration einer singulären Kopie desjenigen Gens, das für eine veränderte Serin-Acetyltransferase kodiert, in das Wirtsgenom.

Beschreibungen und Referenzen für diese Techniken sind in folgenden Publikation zu finden: Shevell et al., 1988, J. Bacteriol. 170: 3294 - 3296; Kulakauskas et al., 1991, J. Bacteriol. 173: 2633 - 2638.

Vorzugsweise werden cysE-Allele unterschiedlichen $K_i$'s auf einen Vektor niederer Kopienzahl kloniert und in den entsprechenden Produktionsstamm transformiert.

Fig. 1 zeigt die Biosynthese von L-Methionin, ausgehend von Homoserin.

Fig. 2 zeigt die Biosynthese von Glutathion, ausgehend von Glutamat.

Fig. 3 zeigt die Biosynthese von Biotin, ausgehend von Dethiobiotin.

Fig. 4 zeigt die Biosynthese von L-Cystein in E. coli, ausgehend von Glucose.

Fig. 5 zeigt die Aminosäureabfolge der Serin-Acetyltransferase aus E. coli.

Fig. 6 zeigt die DNS-Sequenz des E. coli cysE-Gens und die von dieser Sequenz abgeleitete Aminosäuresequenz der Serin-Acetyltransferase.

Fig. 7 zeigt die Restriktionskarte des Plasmids pP1 aus Beispiel 1 enthaltend ein feedbackresistentes cysE-Allel, kloniert als 2.25 kb PvuII-Fragment in pUC19.

Fig. 8 zeigt das Plasmid pPC43 aus Beispiel 2 , enthaltend das cysE-Wildtyp-Gen als 1.15 kb großes EcoRI/BamHI-Fragment in pBluescript.

Fig. 9 zeigt die Plasmidkarte von pACYC184-LH aus Beispiel 3 enthaltend ein feedbackresistentes cysE-Allel mit carboxyterminaler Deletion, und aus Beispiel 4 enthaltend ein feedbackresistentes cysE-Allel mit verschiedenen Basenaustauschen.

Die folgenden Beispiele dienen der weiteren Erläuterung der Erfindung:

**Beispiel 1**

**Isolierung von E. coli-Mutanten mit Cystein-insensitiven Serin-Acetyltransferase-Enzymen**

Durch Reversion auxotropher E. coli-Stämme ist es möglich, Regulationsmutanten zu erzeugen. Mutanten mit den gewünschten Eigenschaften (Cystein-Insensitivität der Serin-Acetyltransferase) werden unter den Revertanten Cystein-auxotropher cysE-E.coli-Stämme gesucht.

Für die Isolierung der Revertanten wurden die Cystein-auxotrophen E. coli-Stämme JM15 (CGSC # 5042: cysE50, tfr-8), und JM39 (CGSC # 5043: cysE51, tfr-8), hinterlegt bei der Deutschen Sammlung für Mikroorganismen in Braunschweig unter der Hinterlegungsnummer DSM 10173 verwendet. Zur Erzeugung von Cystein-prototrophen Revertanten wurden diese Stämme mit dem Mutagen Nitrosoguanidin nach Miller, J. H. (1972), Experiments in Molecular Genetics. Cold Spring Harbor Press: 125 - 129, Cold Spring Harbor Laboratory behandelt. Auf Cystein-freiem Minimalmedium wurde nach Cystein-prototrophen Revertanten gesucht. Etwa 1000 der erhaltenen Revertanten wurden im Kreuzfütterungsexperiment zunächst auf Cysteinausscheidung getestet. Dazu wurden die zu testenden Revertanten auf Cystein-freies Minimalmedium (12 g/L $K_2HPO_4$, 3 g/L $KH_2PO_4$, 5 g/L $(NH_4)_2SO_4$, 0,3 g/L $MgSO_4$ x 7 $H_2O$, 0,015 g/L $CaCl_2$ x 2 $H_2O$, 0,002 g/L $FeSO_4$ x 7 $H_2O$, 1 g/L $Na_3$Citrat x 2 $H_2O$, 0,1 g/L NaCl, 15 g/L Bacto-Agar, 1 ml/L Spurenelementlösung, bestehend aus 0,15 g/L $Na_2MoO_4$ x 2 $H_2O$, 2,5 g/L $H_3BO_3$, 0,7 g/L $CoCl_2$ x 6 $H_2O$, 0,25 g/L $CuSO_4$ x 5 $H_2O$, 1,6 g/L $MnCl_2$ x 4 $H_2O$, 0,3 g/L $ZnSO_4$ x 7 $H_2O$, das mit 1 % Glucose supplementiert und mit 5 x $10^6$ Zellen des Cystein-auxotrophen Indikatorstammes JM39 pro ml beimpft war, ausgebracht und 48 Stunden bei 37 °C inkubiert. Der Radius des Fütterhofes um die Testkolonie (Halo) wurde als semiquantitatives Maß für die Cysteinausscheidung durch den Teststamm genommen. Alle Revertanten, die eine Wachstumszone größer als 2 mm aufwiesen, wurden als positiv eingestuft und nach mehreren Reinigungsausstrichen isoliert und konserviert.

Zur Untersuchung der biochemischen Grundlage für die Cysteinausscheidung der Revertanten wurde die Aktivität der Serin-Acetyltransferase in vitro bestimmt, sowie ihre Hemmbarkeit durch Cystein gemessen. Für die Bestimmung wurden S30-Extrakte (20 min bei 30 000 g und 4°C zentrifugierte Zellaufbrüche) der ausgewählten Revertanten, der Ausgangsstämme sowie des Vergleichsstamms E. coli W3110 (ATTC 27325) verwendet. Es wurde eine Reihe von Revertanten gefunden, deren Serin-Acetyltransferase-Aktivität in Gegenwart verschiedener Konzentrationen des Inhibitors L-Cystein noch eine signifikante Restaktivität aufwies ($K_I$-Wert zwischen 5 und 50 μM).

Zur Bestimmung der Befähigung zur Cysteinausscheidung im Flüssigmedium mittels quantitativer Cysteinbestimmung wurden 50 ausgewählte cysE-Revertanten in 20 ml Standardproduktionsmedium über einen Zeitraum von 48 Stunden bei 30°C und 170 Upm inkubiert. Das Standardproduktionsmedium bestand aus 15 g/L Glucose, 0,08 g/L Bactotrypton, 0,04 g/L Hefeextrakt, 5 mg/L Vitamin B1, 3 g/L $KH_2PO_4$, 12 g/L $K_2HPO_4$, 0,3 g/L $MgSO_4$ x 7 $H_2O$, 0,1 g/L NaCl, 5 g/L $(NH_4)_2SO_4$, 14,7 mg/L $CaCl_2$ x 2 $H_2O$, 2 mg/L $FeSO_4$ x 2 $H_2O$, 1 g/L $Na_3$ Citrat x 2 $H_2O$, 5 g/L $Na_2S_2O_3$ x 5 $H_2O$ und 1 ml/L Spurenelementlösung (vgl. oben). Nach 24 und 48 Stunden wurde jeweils eine Probe (10 μl) entnommen, gegebenenfalls verdünnt, und im zellfreien Überstand die Cysteinkonzentration calorimetrisch nach Gaitonde, M. K. (1967), Biochem. J. 104: 627 - 633, bestimmt. Das Ausmaß der Cysteinausscheidung dieser Mutanten variierte von 5-60 mg/L Cystein im Kulturüberstand. Im E.coli-Wildtypstamm hingegen konnte vergleichsweise keinerlei Cysteinausscheidung nachgewiesen werden. Aus diesem Screening wurden 8 Revertanten ausgewählt, deren Cysteinausscheidung zwischen 40 und 60 mg/L lag.

Für die exakte Analyse der genetischen Grundlage der Endprodukt-Resistenz der Serin-Acetyltransferasen dieser 8 Mutanten wurden deren cysE-Strukturgene kloniert und deren DNS-Sequenz bestimmt.

Da die DNS-Sequenz des cysE-Wildtyp-Gens sowie die chromosomale Restriktionskarte der das cysE-Gen flankierenden Regionen von E. coli bekannt ist (Denk und Böck, 1987, J. Gen. Microbiol. 133: 515 - 525), weiß man, daß das cysE-Strukturgen auf einem 2,25 kb großen PvuII-DNS-Fragment liegt.

Zur Klonierung der für die Cystein-insensitiven Serin-Acetyltransferasen kodierenden cysE-Gene wurde die chromosomale DNS der selektierten Revertanten vollständig mit PvuII hydrolysiert, das DNS-Hydrolysat über ein präparatives Agarosegel aufgetrennt und die DNS im Größenbereich von 2 - 3 kb isoliert. Das isolierte PvuII-Hydrolysat wurde mit dem SmaI-linearisierten und mit alkalischer Phosphatase dephosphorylierten Plasmid-Vektor pUC19 (erhältlich bei der Fa. Boehringer Mannheim) mittels T4 DNS-Ligase verknüpft.

Der Cystein-auxotrophe cysE-Stamm JM15 (CGSC#5042) wurde mit dem jeweiligen Ligationsgemisch transformiert und die Selektion auf cysteinfreiem, ampicillinhaltigem (50 mg/L) Minimalmedium vorgenommen. Die selektierten und die die Cystein-Auxotrophie des Wirtsstammes komplementierenden Plasmide (vgl. Fig. 7) wiesen im Restriktionsmuster das für das cysE-Gen erforderliche Spaltmuster auf (Denk und Böck, 1987, J. Gen. Microbiol. 133: 515 - 525). Auch im Kreuzfütterungstest verursachten die selektierten Transformanten ein intensives Wachstum des Indikatorstammes JM35 (Halo > 4 mm). Die Aktivitätsbestimmung der Serin-Acetyltransferase in Zellextrakten dieser cysE-Mutanten, die durch 20 min Zentrifugation bei 30 000 g und 4°C gewonnen wurden, ergab eine reduzierte Sensitivität gegenüber L-Cystein. Zur exakten Identifizierung der zur Endprodukt-Resistenz führenden Veränderungen im Strukturgen der einzelnen cysE*-Allele wurde deren DNS unter Verwendung cysE-Gen spezifischer Oligonukleotide sequenziert und die ermittelten Nukleotidsequenzen mit der des cysE-Wildtypgens verglichen. Dieser Vergleich der Nukleotidsequenzen ergab die in folgender Tabelle 2 zusammengefaßten Unterschiede zur DNS- und Aminosäuresequenz der Wildtypform (vgl. Fig. 5 und 6).

Tab.2: Feedbackresistente cysE-Allele, entstanden durch chemische Mutagenese

| cysE-Mutante | Nukleotid-Austausch (Nr.) | Aminosäure-Austausch (Nr.) | $K_i$ ($\mu$M) |
|---|---|---|---|
| cysEII | GGC->AGC (934) | Gly238->Ser238 | 10 |
| cysEIII | GGT->GAT (716) | Gly165->Asp165 | 10 |
| cysEVII | GCT->GTT (932) | Ala237->Val237 | 10 |
| cysEX | ACG->GCG (721) | Thr167->Ala167 | 50 |
| cysEXI | GGT->AGT (955) | Gly245->Ser245 | 700 |
| | ACG->GCG (721) | Thr167->Ala167 | |
| cysEXII | AAA->CAA (511) | Lys97->Gln97 | 40 |
| | GGC->AGC (934) | Gly238->Ser238 | |
| | TTT->TTG (1023) | Phe267->Leu267 | |
| cysEXIII | GTT->GCT (713) | Val164->Ala164 | 30 |
| | TTT->TTG (1023) | Phe267->Leu267 | |
| cysEXVI | GAT->GGT (971) | Asp250->Gly250 | 50 |
| | AAG->TAG (973) | Lys251->Stop251 | |

**Beispiel 2**

**Erzeugung von Endprodukt-insensitiven Serin-Acetyltransferasen durch gezielte Basenaustausche im cysE-Strukturgen**

In Beispiel 1 wurden insgesamt 8 verschiedene cysE-Allele beschrieben, die aufgrund von Basenaustauschen und damit einhergehender Aminosäureveränderungen eine beträchtliche Insensitivierung der Serin-Acetyltransferase gegenüber dem Inhibitor L-Cystein aufweisen. Diese veränderten Enzyme unterscheiden sich nicht nur in der Position der zur Resistenz führenden Aminosäureaustausche, sondern teilweise auch im Maß der Sensitivität gegenüber dem Inhibitor L-Cystein. Durch ortsspezifische Mutagenese wurden endprodukt-resistente Serin-Acetyltransferase-Enzyme mit neuen Eigenschaften konstruiert, in welche die in Beispiel 1 beschriebenen Aminosäureaustausche untereinander kombiniert wurden. Die dafür notwendigen Mutagenesen wurden gemäß dem Stand der Technik nach der von Kunkel et al. (1987), Meth. Enzymol. 154: 367 - 382, beschriebenen Methode durchgeführt.

Als Ausgangsplasmid für die Mutagenesen wurde das in Fig. 8 dargestellte cysE-Plasmid pPC43 (hinterlegt bei der Deutschen Sammlung für Mikroorganismen, Braunschweig, unter der Hinterlegungsnummer DSM 10171) verwendet, welches das 1,15 kb große cysE-Wildtypgen in der EcoRI-BamHI-Stelle des Phagemid-Vektors pBluescriptII SK+ (Fa. Stratagene, Heidelberg) enthält. Dieses cysE-WT-Gen wurde nach der Methode der Polymerasekettenreaktion (PCR) (Saiki et al. 1988, Science 239: 487 - 491) aus der genomischen DNS des E. coli Wildtypstammes W3110 (ATTC 27325) amplifiziert. Verwendet wurden die Oligonukleotide cysE-fw1 (SEQ ID NO: 3) ("sense-Primer") und cysE-rev1 (SEQ ID NO: 4) ("antisense-Primer"). Die Nukleotidsequenz dieser Primer setzt sich wie folgt zusammen:

<u>cysE</u>-fw1: (SEQ ID NO: 3)


5'-GCCT<u>GGATCC</u>TGCAGTCGAC**CTGGCGCATCGCTTCGGCGTTG**-3'


der fett markierte Anteil entspricht Basen 9-30 aus der cysE-DNS-Sequenz von Fig. 6, unterstrichen ist die inkorporierte BamHI-Stelle.


<u>cysE</u>-rev1: (SEQ ID NO: 4)


5'-GTAGGAGCTCTGCA<u>GAATTC</u>**GGGTATCCGGGAGCGGTATTG**-3',


der fett markierte Anteil entspricht Basen 1106-1126 aus der cysE-DNS-Sequenz von Fig. 6, unterstrichen ist die inkorporierte EcoRI-Stelle.

Die PCR-Experimente wurden in 30 Zyklen in Gegenwart von 200 µM Deoxynukleotidtriphosphaten (dATP, dCTP, dGTP, dTTP), je 1 µM des entsprechenden Oligonukleotids, 100 ng W3110-DNS, Reaktionspuffer (10 mM Tris-HCl pH 8,3, 50 mM KCl, 1,5 mM $MgCl_2$, 0.01 % Gelatine) und 5 Einheiten einer hitzestabilen Vent-DNS-Polymerase (Fa.Biolabs) in einem Thermocycler (Gene-ATAQ-Controler, Fa.Pharmacia) unter folgenden Bedingungen durchgeführt: 96 °C, 1,5 min; 62 °C, 1 min; 72 °C, 3 min. Das Amplifikationsprodukt wurde mit BamHI und EcoRI hydrolysiert, über ein Agarosegel gereinigt und als 1,15 kb großes DNS-Fragment in den mit BamHI und EcoRI linearisierten Phagemid-Vektor BluescriptII SK+ kloniert, wobei das <u>cysE</u>-Plasmid pPC43 entstand (Fig. 8).

Die gewünschten Mehrfachmutanten wurden nach folgender Vorgehensweise erstellt:

1) Herstellung des cysE-IV-Allels: Doppelmutante $Val_{237}$ + $Ser_{238}$
Ausgehend vom <u>cysE</u>-Wildtyp-Plasmid pPC43 wurde durch ortsspezifische Mutagenese unter Verwendung des Mutationsoligonukleotids <u>cysE</u>-Mut-1 (SEQ ID NO: 5) (Tab. 3) zunächst an Position 238 an Stelle des Glycins ein Serin und in die dabei erzeugte cysE-Mutante pPC34 unter Verwendung des Mutationsoligonukleotids <u>cysE</u>-Mut-3 (SEQ ID NO: 6) (Tab. 3) an Position 237 anstelle des Alanins ein Valin eingeführt, wobei das <u>cysE</u>-IV-Allel entstand.

2) Herstellung des cysE-VIII-Allels: Doppelmutante $Val_{237}$ + $Ile_{256}$
Ausgehend vom cysE-Wildtyp-Plasmid pPC43 wurde durch ortsspezifische Mutagenese unter Verwendung des Mutationsoligonukleotids <u>cysE</u>-Mut-6 (SEQ ID NO: 7) (Tab. 3) an Position 256 für das Methionin ein Isoleucin eingeführt, wobei das cysEI-Allel entstand. In dieses wurde mittels Mutationsoligunukleotid cysE-Mut-3 (SEQ ID NO: 6) (Tab. 3) anstelle des Alanins an Position 237 ein Valin eingeführt. Dabei entstand das Allel cysE-VIII.

3) Herstellung des CysE-VI-Allels: Doppelmutante $Ser_{238}$ + $Ile_{256}$
In das cysE-I-Allel (Mutante $Ile_{256}$) wurde mit Hilfe des Mutationsoligonukleotids <u>cysE</u>-Mut-1 (SEQ ID NO: 5) (Tab. 3) an Position 238 anstelle des Glycins ein Serin eingeführt, wobei das cysE-VI-Allel entstand.

4) Herstellung des cysE-V-Allels: Dreifachmutante $Val_{237}$ + $Ser_{238}$+ $Ile_{256}$
In dem cysE-IV-Allel (Doppelmutante $Val_{237}$ + $Ser_{238}$) wurde mit Hilfe des Mutationsoligonukleotids <u>cys</u>E-Mut-6 (SEQ ID NO: 7) (Tab. 3) das Methionin an Position 256 durch ein Isoleucin ersetzt. Dabei entstand das cysE-V-Allel.

5) Herstellung des cysE-XIV-Allels: Doppelmutante $Ala_{167}$ + $Stop_{251}$
Ausgehend vom Allel cysE-Del255 (vgl. Beispiel 3) wurde unter Verwendung des Mutationsoligonukleotids cysE-Mut-10 (SEQ ID NO: 8) (Tab. 3) an Position 167 anstelle des Threonins ein Alanin eingeführt, wobei das cysE-XIV-Allel entstand.

6) Herstellung des cysE-XVII-Allels: Doppelmutante $Asp_{165}$ + Ala167

Ausgehend von cysE-III-Allel (Mutante $Asp_{165}$, vgl. Beispiel 1) wurde mit Hilfe des Oligonukleotids cysE-Mut-10 (SEQ ID NO: 8) (Tab. 3) die Aminosäure Threonin an Position 167 durch ein Alanin ersetzt. Hierbei entstand das Allel cysEXVII.

7) Herstellung des cysE-XXIII-Allels: Dreifachmutante $Ala_{167}$ +$Val_{237}$ + $Ser_{238}$

In das cysE-IV-Allel (Doppelmutante $Val_{237}$+$Ser_{238}$) wurde unter Verwendung des Mutationsoligonukleotids cysE-Mut-10 (SEQ ID NO: 8) (Tab. 3) an Position 167 anstelle des Threonins ein Alanin eingeführt, wobei das cysE-XXIII-Allel entstand.

Die Korrektheit der eingeführten Mutationen wurde durch DNS-Sequenzanalyse des gesamten Strukturgens der jeweiligen Mutante überprüft. Eine Übersicht der cysE*-Mehrfachmutanten findet sich in Tab. 4.

Für die Bestimmung der biochemischen Parameter wie Enzymaktivität und Hemmkonstante $K_i$ wurde analog zu der Beschreibung in Beispiel 1 vorgegangen.

Tab. 3: Oligonukleotide, verwendet für die ortsspezifische Mutation zur Erzeugung neuer feedbackresistenter cysE-Allele

| SEQ ID NO: | Mutations-oligonu-kleotid | Nukleotidsequenz | Position in der Fig. 6 | Aminosäure-austausch |
|---|---|---|---|---|
| 5 | cysE-Mut-1 | 5'-GCCGCTAGCGTTCCGGCT-3' | 928-945 | Gly238->Ser238 |
| 6 | cysE-Mut-3 | 5'-CCGCCGCATACCACCGCCGTT-3' | 913-933 | Ala237->Val237 |
| 7 | cysE-Mut-6 | 5'-CCATCAATGGATATAGACCAGCAT-3 | 976-999 | Met256->Ile256 |
| 8 | cysE-Mut-10 | 5'-GTCGTTGGTGAAGCGGCGGTGATT-3' | 709-732 | Thr167->Ala167 |

EP 0 858 510 B1

Tab. 4: Feedbackresistente cysE-Allele, entstanden durch gezielte ortsspezifische Mutagenese

| cysE-Mutante | Nukleotid-Austausch (Nr.) | Aminosäure Austausch (Nr.) | $K_i(\mu M)$ |
|---|---|---|---|
| cysEIV | GCT->CTT(932) | Ala237->Val237 | 40 |
| | GGC->AGC(934) | Gly238->Ser238 | |
| cysEV | GCT->GTT(932) | Ala237->Val237 | 10 |
| | GGC->AGC(934) | Gly238->Ser238 | |
| | ATG->ATA(990) | Met256->Ile256 | |
| cysEVI | GGC->AGC(934) | Gly238->Ser238 | 10 |
| | ATG->ATA(990) | Met256->Ile256 | |
| cysEVIII | GCT->GTT(932) | Ala237->Val237 | 30 |
| | ATG->ATA(990) | Met256->Ile256 | |
| cysEXIV | ACG->GCG(721) | Thr167->Ala167 | >1000 |
| | ATG->TAG(988+989) | Met256->Stop256 | |
| cysEXVII | GGT->GAT(716) | Gly165->Asp165 | 100 |
| | ACG->GCG(721) | Thr167->Ala167 | |
| cysEXXIII | ACG->GCG(721) | Thr167->Ala167 | 2300 |
| | GCT->GTT(932) | Ala237->Val237 | |
| | GGC->AGC(934) | Gly238->Ser238 | |

**Beispiel 3**

**Erzeugung von Endprodukt-insensitiven Serin-Acetyltransferasen durch kontrollierte Verkürzung des Carboxy-Terminus des Enzyms mittels PCR**

Gezielte Veränderungen von einzelnen oder mehreren Aminosäuren innerhalb eines Proteins sind Stand der Technik und lassen sich mittels PCR-Technologie (Saiki et al., 1988, Science 239: 487 - 491) unter Verwendung geeigneter Mutationsprimer auf DNS-Ebene gut durchführen. Für die Expression der veränderte Proteine werden die erhaltenen PCR-Produkte in ein geeignetes Plasmid-/Wirtssystem kloniert.

Unter Verwendung der in Tab. 5 dargestellten Oligonukleotid-Primer wurden aus der genomischen DNS des E.coli Wildtypstammes W3110 (ATTC 27325) cysE-Mutanten mit carboxyterminalen Deletionen unterschiedlicher Länge hergestellt, die in Tab. 6 zusammengestellt sind.

Die PCR-Experimente wurden in 30 Zyklen in Gegenwart von 200 μM Deoxynukleotidtriphosphaten (dATP, dCTP, dGTP, dTTP), je 1 μM der Oligonukleotide des "sense-Primer" cysE-LHfw1 (SEQ ID NO: 9) und dem entsprechenden "antisense-Primer" (SEQ ID NO: 10-23) (Tab. 5), 100 ng W3110-DNS, Reaktionspuffer (10 mM Tris-HCl pH 8,3, 50 mM KCl, 1,5 mM MgCl$_2$, 0,01 % Gelatine) und 5 Einheiten einer hitzestabilen Vent-DNS-Polymerase (Fa. Biolabs) in einem Thermocycler (Gene-ATAQ-Controler, Fa. Pharmacia) unter folgenden Bedingungen durchgeführt: 96 °C, 1,5 min; 62 °C, 1 min; 72 °C, 3 min.


<pre>
     cysE-LHfw1 (SEQ ID NO: 9)
</pre>


<pre>
  5'-TGGA<u>CCAGAGCTCTGG</u>**CTGGCGCATCGCTTCGGCGTTG**-3'
</pre>


Der fettmarkierte Anteil entspricht Basen 9-30 der cysE-Sequenz von Fig. 6, unterstrichen ist die inkorporierte BstXI/SacI-Stelle.

Das nach Amplifikation entstandene Produkt wurde mit den Enzymen SacI und NsiI hydrolysiert, anschließend über ein Agarosegel gereinigt und das jeweils isolierte cysE-DNA-Fragment in den mit SacI und NsiI linearisierten Vektor pACYC184-LH /DSM 10172) (vgl. Fig. 9) ligiert. Der jeweilige Ligaseansatz wurde in den Cystein-auxotrophen cysE-Stamm JM15 (CGSC#5042) transformiert und die Selektion auf Cystein-freiem, Tetracyclin-haltigem (20 mg/l) Minimalmedium vorgenommen.

Die aus dieser Klonierung hervorgegangenen Plasmide wurden entsprechend ihres Deletionsausmaßes als pACYC184/cysE-Del bezeichnet (vgl. Fig. 9 zur Plasmidkarte). Die Bestimmung der enzymatischen Aktivität und der Hemmkonstante K$_i$ sowie der Kreuzfütterungstest wurden analog der Beschreibung in Beispiel 1 vorgenommen. Die Korrektheit der eingeführten Deletionen wurde durch DNS-Sequenzanalyse bestätigt.

Die Ergebnisse dieser Untersuchungen sind in Tab. 6 zusammengestellt.

Tab.5 Antisense-Oligonukleotide für die Herstellung von cysE-Allelen mit carboxyterminalen Deletionen

| SEQ ID NO | Mutations-oligonukleotid | Nukleotidsequenz | Position in der Fig. 6 |
|---|---|---|---|
| 10 | cysE-Del270 | 5'-CTCGATGCATTACGTATTACCCATACTCAAATCTATGGTTAATACC-3' | 1006-1032 |
| 11 | cysE-Del268 | 5'-CTCGATGCATTACGTATTACTCAAATGTATGGTTAATACCGTTGAA-3' | 1000-1026 |
| 12 | cysE-Del263 | 5'-CTCGATGCATTACGTATTAAATACCGTTGAAATGCTGGTCCATATC-3' | 985-1011 |
| 13 | cysE-Del259 | 5'-CTCGATGCATTACGTATTAATGCTGGTCCATATCCATTGATGGCTT-3' | 973- 999 |
| 14 | cysE-Del258 | 5'-CTCGATGCATTACGTATTACTGGTCCATATCCATTGATGGCTTATC-3' | 970- 996 |
| 15 | cysE-Del257 | 5'-CTCGATGCATTACGTATTAGTCCATATCCATTGATGGCTTATCGCTG-3' | 966- 993 |
| 16 | cysE-Del256 | 5'-CTCGATGCATTACGTATTACATATCCATTGATGGCTTATCGCTGTC-3' | 964- 990 |
| 17 | cysE-Del255 | 5'-CTCGATGCATTACGTATTAATCCATTGATGGCTTATCGCTGTCTGG-3' | 961- 987 |
| 18 | cysE-Del250 | 5'-CTCGATGCATTACGTATTAATCGCTGTCTGGTTTACCGACAATACG-3' | 946- 972 |
| 19 | cysE-Del249 | 5'-CTCGATGCATTACGTATTAGCTGTCTGGTTTACCGACAATACGAGC-3' | 943- 969 |
| 20 | cysE-Del248 | 5'-CTCGATGCATTACGTATTAGTCTGGTTTACCGACAATACGAGCCGG-3' | 940- 966 |
| 21 | cysE-Del245 | 5'-CTCGATGCATTACGTATTAACCGACAATACGAGCCGGAACGCCAGC-3' | 931- 957 |
| 22 | cysE-Del239 | 5'-CTCGATGCATTACGTATTAAACGCCAGCGGCGGTGGTATCCGGCGG-3' | 913- 939 |
| 23 | cysE-Del227 | 5'-CTCGATGCATTACGTATTACAGCACCACGGAACCTGCGCCAATCTT-3' | 877- 903 |

EP 0 858 510 B1

Der fett markierte Anteil entspricht den jeweiligen Basen in der Sequenz der Fig. 6, unterstrichen ist die NsiI-Stelle

```
Tab. 6: Feedbackresistente cysE-Allele, entstanden durch
carboxyterminale Deletionen
```

| cysE-Mutante | Anzahl deletier-ter Aminosäuren | Terminale Aminosäuren | $K_i$ ($\mu$M) |
|---|---|---|---|
| cysE-Del259 | 14 | His259 | 7,5 |
| cysE-Del258 | 15 | Gln258 | 5 |
| cysE-Del257 | 16 | Asp257 | 7,5 |
| cysE-Del256 | 17 | Met256 | 12,5 |
| cysE-Del255 | 18 | Asp255 | 30 |
| cysE-Del250 | 23 | Asp250 | 20 |
| cysE-Del249 | 24 | Ser249 | 15 |
| cysE-Del248 | 25 | Asp248 | 12,5 |

**Beispiel 4**

**Transformation eines E. coli-Wirtsstammes mit veränderten Serin-Acetyltransferasen zur Überproduktion von L-Cystein bzw. L-Cystein-verwandten Produkten in Schüttelkolben**

Für die Produktion wurde der Vektor pACYC184-LH, der sich durch eine niedrige Kopienzahl auszeichnet, verwendet. Hierfür wurden die cysE-Gene auf den Plasmiden aus den Beispielen 1 und 2 durch PCR amplifiziert.

Die PCR-Experimente wurden in 30 Zyklen in Gegenwart von 200µM Deoxynukleotidtrphosphaten (dATP, dCTP, dGTP, dTTP), je 1 µM der Oligonukleotide des "sense-Primer" cysE-LHfw1 (SEQ ID NO: 9) und des entsprechenden "antisense Primer" cysE-LHrev1 (SEQ ID NO: 24), 10 ng jeweilige Plasmid-DNA, Reaktionspuffer (10 mM Tris-HCl pH 8,3, 50 mM KCl, 1,5 mM MgCl$_2$, 0,01 % Gelatine) und 5 Einheiten einer hitzestabilen Vent-DNA-Polymerase (Fa. Biolabs) in einem Thermocycler (Gene-ATAQ-Controler, Fa. Pharmacia) unter folgenden Bedingungen durchgeführt: 96°C, 1,5 min; 62°C, 1 min; 72°C, 3 min.

```
cysE-LHfw1 (SEQ ID NO: 9)


5'-TGGACCAGAGCTCTGGCTGGCGCATCGCTTCGGCGTTG-3'
```

Die unterstrichenen Basen entsprechen den inkorporierten Restriktionsschnittstellen BstXI und SacI, die restlichen, fett gedruckten Basen entsprechen Position 9-30 der cysE-Sequenz von Fig. 6.

```
cysE-LHrev1 (SEQ ID NO: 24),


5'-CTCGATGCATTACGTAGGGGTATCCGGGAGCGGTATTG-3'
```

Die unterstrichenen Basen entsprechen der inkorporierten Restriktionsschnittstellen NsiI, die restlichen, fett gedruckten Basen entsprechen Position 1106 - 1127 der cysE-Sequenz von Fig. 6.

Das nach der Amplifikation entstandene Produkt wurde mit den Enzymen SacI und NsiI hydrolysiert, anschließend über ein Agarosegel gereinigt und das jeweils isolierte cysE-DNA-Fragment in den mit SacI und NsiI linearisierten Vektor pACYC184-LH (DSM 10172) ligiert.

Der jeweilige Ligaseansatz wurde in den cysteinauxotrophen cysE-Stamm JM15(CGSC#5042) transformiert und die Selektion auf cysteinfreiem, tetracyclinhaltigem (20 mg/L) Minimalmedium vorgenommen. Die aus dieser Klonierung hervorgegange Reihe an feedbackresistenten cysE-Plasmiden wurde als pACYC184/cysE (vgl. Fig. 9) bezeichnet, wobei jeder Klon mit der entsprechenden cysE-Allelnummer versehen wurde.

Die Bestimmung der enzymatischen Aktivität, der Hemmkonstante $K_i$ sowie der Kreuzfütterungstest wurden analog der Beschreibung in Beispiel 1 vorgenommen.

Zur Bestimmung der Produktionskapazität in Flüssigmedium wurden 20 ml des Standardproduktionsmediums mit einer Einzelkolonie beimpft und 48 Stunden bei 30°C und 170 Upm inkubiert. Das Produktionsmedium bestand aus 15 g/L Glucose, 0,08 g/L Bactotrypton, 0,04 g/L Hefeextrakt, 5 mg/L Vitamin B1, 3 g/L $KH_2PO_4$, 12 g/L $K_2HPO_4$, 0,3 g/L $MgSO_4$ x 7 $H_2O$, 0,1 g/L NaCl, 5 g/L $(NH_4)_2SO_4$, 14,7 mg/L $CaCl_2$ x 2 $H_2O$, 2 mg/L $FeSO_4$ x 2 $H_2O$, 1 g/L $Na_3$ Citrat x 2 $H_2O$, 5 g/L $Na_2S_2O_3$ x 5 $H_2O$, 1 ml/L Spurenelementlösung, 0,025 mg/L Tetracyclin. Die Spurenelementlösung setzte sich aus 0,15 g/L $Na_2MoO_4$ x 2 $H_2O$, 2,5 g/L $H_3BO_3$, 0,7 g/L $CoCl_2$ x 6 $H_2O$, 0,25 g/L $CuSO_4$ x 5 $H_2O$, 1,6 g/L $MnCl_2$ x 4 $H_2O$ und 0,3 g/L $ZnSO_4$ x 7 $H_2O$ zusammen. Nach 24 und 48 Stunden wurde jeweils eine Probe (10 µl) entnommen, entsprechend verdünnt und im zellfreien Überstand die Produktkonzentration calorimetrisch nach Gaitonde, M. K. (1967), Biochem. J. 104, 627-633, bestimmt. Für den mit unterschiedlichen cysE-Mutanten transformierten Produktionsstamm JM15 wurden dabei Konzentrationen zwischen 50 und 300 mg/L an Cystein gemessen.

**Beispiel 5**

**Konstruktion chromosomal kodierter, feedbackresistenter cysE-Allele mit Hilfe eines rekombinanten λ-Prophagen und Produktion von L-Cystein oder L-Cystein-abgeleiteten Produkten im 1 L-Fermenter**

Zur Integration in die chromosomale "attachment site" (attλ) wurden die cysE-Allele cysEIV, cysEX und cysEXI in das Plasmid pRS551 (Simons et al., 1987, Gene **53**: 85-96) kloniert. Dazu wurde das jeweilige cysE-Allel durch PCR aus dem entsprechenden cysE-Plasmid amplifiziert. Die verwendeten Oligonukleotide cysE-fw1 und cysE-rev1 sind in Beispiel 1 beschrieben. Die Amplifizierung erfolgte wie in Beispiel 3 beschrieben. Die erhaltenen Fragmente wurden mit EcoRI/BamHI gespalten, über ein Agarosegel gereinigt und in den EcoRI/BamHI gespaltenen Vektor pRS551 ligiert. Hierbei entstanden die auf dem Vektor pRS551 basierenden rekombinanten Plasmide pRScysEIV, X und XI.

Durch die Herstellung eines Plattenlysats auf einem pRScysE-tragenden $recA^+$-Stamm (z.B. YMC9, ATCC 3397) mit dem λRS45-Phagen wurde in vivo durch homologe Rekombination ein heterogenes lambda-Lysat erzeugt, das neben λRS45-Phagen auch rekombinante cysE-Allele-tragende λRS45-Derivate enthielt (Simons et al., 1987, Gene 53: 85-96).

Zur Selektion auf die rekombinanten RS45-Derivate wurde der cysE-Stamm JM15 verwendet, der mit dem heterogenen lambda-Lysat infiziert und anschließend auf Kanamycin-haltigen (25 mg/L) LB-Platten plattiert wurde. Die erhaltenen lysogenen, Kanamycin-resistenten Klone wurden dann auf ihre Fähigkeit getestet, auf Minimalmediumplatten ohne Cystein zu wachsen. Ein jeweils Cystein-prototropher Klon wurde ausgewählt und für die Herstellung eines homogenen cysE-λ-Lysats (durch UV-Induktion, Simons et al., 1987, Gene 53: 85-96) verwendet.

Mit diesen jeweils erhaltenen homogenen cysE-λ-Lysaten wurde der Stamm JM 15 infiziert. Die daraus hervorgegangenen Stämme JM15attλ::cysE, wurden, wie in Beispiel 6 beschrieben, fermentiert. Die jeweiligen Medien enthielten anstelle von Tetracyclin als Selektionsagens jeweils 25 mg/L Kanamycin.

Die Ausbeuten an Cystein lagen mit cysEIV bei 0,5, mit cysEX bei 1,8 und mit cysEXI bei 2,1 g/L (vgl. Tab. 7).

**Beispiel 6**

**Einfluß von verschiedenen, plasmidkodierten cysE-Allelen auf die Produktion von L-Cystein oder L-Cystein-abgeleiteten Produkten im 1 L-Fermenter unter Verwendung des Wirtsstammes JM15**

20 mL, in einem 100 mL Erlenmeyerkolben befindliches LB Medium (1% Trypton, 0,5% Hefeextrakt, 0,5% NaCl) wurden mit einer Einzelkolonie des mit dem jeweiligen cysE-Plasmid transformierten Produktionsstammes JM15 (CGSC#5042) beimpft.

Nach 7-stündiger Inkubation im Bakterienschüttler (150 rpm, 30°C) wurden die jeweiligen Vorkulturen in 100 mL SM1-Medium überführt. Das SM1-Medium enthielt 5 g/L Glucose, 5 mg/L Vitamin B1, 3 g/L $KH_2PO_4$, 12 g/L $K_2HPO_4$, 0,3 g/L $MgSO_4$ x 7 $H_2O$, 0,1 g/L NaCl, 5 g/L $(NH_4)_2SO_4$, 14,7 mg/L $CaCl_2$ x 2 $H_2O$, 2 mg/L $FeSO_4$ x 2 $H_2O$, 1 g/L $Na_3$Citrat x 2 $H_2O$, 1 ml/L Spurenelementlösung, 25 mg/L Tetracyclin. Die Spurenelementlösung setzte sich zusammen aus 0,15 g/L $Na_2MoO_4$ x 2 $H_2O$, 2,5 g/L $H_3BO_3$, 0,7 g/L $CoCl_2$ x 6 $H_2O$, 0,25 g/L $CuSO_4$ x 5 $H_2O$, 1,6 g/L $MnCl_2$ x 4 $H_2O$ und 0,3 g/L $ZnSO_4$ x 7 $H_2O$. Die Kulturen wurden in 1-L-Erlenmeyerkolben bei 30° C für 17 h mit 150 rpm geschüttelt. Nach dieser Inkubation betrug die $OD_{600}$ zwischen 4 und 5. Die weitere Fermentation wurde in Forschungsfermentern BIOSTAT M der Firma Braun-Melsungen durchgeführt. Ein Kulturgefäß mit 2 L Gesamtvolumen wurde benutzt.

Das Fermentationsmedium enthielt 15 g/L Glucose, 5 g/L NaCl, 0,3 g/L $MgSO_4$ x 7 $H_2O$, 15 mg/L $CaCl_2$ x 2 $H_2O$, 75 mg/L $FeSO_4$ x 7 $H_2O$, 1 g/L $Na_3$Citrat x 2 $H_2O$, 1,5 g/L $KH_2PO_4$, 1 ml Spurenelementlösung(siehe oben), 5 mg/L Vitamin B1, 2,5 g/L Hefeextrakt (Difco), 2,5 g/L Trypton (Difco) und 25 mg/L Tetracyclin.

Die Glucosekonzentration im Fermenter wurde zu Beginn durch Zupumpen einer 700 g/L (w/v) Glucoselösung (sterilisiert) auf einen Wert von 15 g/L und der pH-Wert durch Zupumpen von 25% $NH_4OH$-Lösung auf 7,0 eingestellt. Nach Erreichen einer $OD_{600}$ von 10 wurde aus einer sterilen 100 g/L (w/v) Thiosulfat-Stammlösung 300 mg pro Stunde zugefüttert. 100 mL Vorkultur wurden zum Animpfen in das Fermentergefäß gepumpt. Das Anfangsvolumen betrug ca. 1 L. Die Kulturen wurden zunächst mit 400 rpm gerührt und mit 1,5 vvm einer über einen Sterilfilter entkeimten Preßluft begast. Die Fermentation wurde bei einer Temperatur von 30°C durchgeführt.

Der pH-Wert wurde durch automatische Korrektur mit 25 % $NH_4OH$ auf einem Wert von 7,0 gehalten. Die Sauerstoffsättigung in der Fermentationsbrühe sollte zu keinem Zeitpunkt der Fermentation unter 20 % abfallen, sie wurde über die Rührgeschwindigkeit kontrolliert. In zwei- bis dreistündigem Abstand wurden der Glukosegehalt der Nährlösung, die optische Dichte und der Cysteingehalt ermittelt. Die Bestimmung des Glukosegehalts erfolgte enzymatisch mit Hilfe eines Glukoseanalysators der Firma YSI. Die Glukosekonzentration wurde durch kontinuierliches Zufüttern zwischen 10 und 20 g/L eingestellt.

Der Produktgehalt des Mediums wurde aus dem zellfreien Überstand der Probe calorimetrisch nach Gaitonde, M. K. (1967), Biochem. J. 104, 627 - 633, bestimmt.

Nach 44-50 h wurde die Fermentation abgebrochen. Die produzierten Cysteinmengen in g/L nach 48 h sind in Tabelle 7 zusammengefaßt.

Tab. 7: Cysteinausbeute des Produktionsstammes JM 15, transformiert mit unterschiedlichen cysE-Allelen (1L Fermenter)

| cysE-Allel | Cysteinausbeute [g/L] [48 h] |
|---|---|
| pACYC184/cysEIV | 1,6 |
| pACYC184/cysEV | 1,3 |
| pACYC184/cysEVI | 1,4 |
| pACYC184/cysEX | 3,4 |
| pACYC184/cysEXI | 3,4 |
| pACYC184/cysEXII | 1,2 |
| pACYC184/cysEXIV | 2,3 |
| pACYC184/cysEXV | 3,0 |
| pACYC184/cysEXVI | 2,2 |
| pACYC184/cysEXXIII | 2,7 |
| pACYC184/cysEDel255 | 3,9 |

**Beispiel 7**

**Produktion von L-Cystein oder L-Cystein abgeleiteten Produkten mit Corynebakterien**

Die feedbackresistenten cysE-Allele cysEIV, cysEX, cysEXI und cysEXIV (vgl. Tab. 2 in Beispiel 1) wurden mit den Restriktionsenzymen BamHI und EcoRI (Fa. Boehringer Mannheim) aus ihren entsprechenden Plasmiden gespalten und das jeweils 1,15 kb große DNS-Fragment über ein Agarosegel gereinigt und isoliert. Das jeweilige DNS-Fragment wurde durch Einwirkung des Klenow-Fragments der DNS-Polymerase I aus E. coli (Fa. Boehringer Mannheim) glattendig gemacht. Der Vektor pWST1 wurde mit dem Restriktionsenzym SmaI (Fa. Boehringer Mannheim) hydrolysiert und mit dem glattendigen DNS-Fragment mittels T4 DNS-Ligase verknüpft. Der Vektor pWST1 ist ein E. coli/Corynebakterien-Schaukelvektor und kann sowohl in E. coli als auch in Corynebakerien replizieren. Das corynebakterielle Replikon dieses Vektors stammt aus dem Stamm Corynebacterium glutamicum ATCC 19223. Die Herstellung des Vektors pWST1 ist in US-A-4,965,197 beschrieben. Der Ligationsansatz wurde benutzt, um die für Cystein auxotrophe Mutante JM15 zu transformieren. Die komplementierenden Plasmide wurden entsprechend ihrer insertierten cysE-Allele pWST1-cysEIV, pWST1-cysEX, pWST1-cysEXI und pWST1-cysEXIV bezeichnet.

Die pWST1-cysE-Plasmide wurden benützt, um das Corynebacterium glutamicum ATCC21851 zu transformieren. Die Transformation erfolgte über Elektroporation nach der bei Liebl, W. et al., 1989, FEMS Microbiol. Letters, 65, 299-304 ausführlich geschilderten Technik. Die Selektion der rekombinanten Klone erfolgte über die plasmidkodierte Kanamycinresistenz auf Agarplatten mit 25 mg/L Kanamycin.

Die Fermentation erfolgte analog den in Beispiel 6 beschriebenen Bedingungen mit der Ausnahme, daß anstelle von Tetracyclin Kanamycin in der Konzentration von 50 mg/L als Selektionsantibiotikum verwendet wurde.

In der Fermentation zeigte sich, daß der Stamm, der das cysEXI-Allel auf einem Plasmid trägt, die höchsten Cystein-Ausbeuten erreicht.

**Patentansprüche**

1. Serin-Acetyltransferase, die eine im Vergleich zum Wildtyp-Enzym reduzierte Sensitivität gegenüber dem Inhibitor L-Cystein aufweist und deren Proteinsequenz im Vergleich zur Wildtyp-Sequenz mindestens eine Mutation oder Deletion aufweist, **dadurch gekennzeichnet, daß** die Mutation im Sequenzbereich von Aminosäure in Position 97 bis einschließlich der Aminosäure in Position 273 liegt oder die Deletion im carboxyterminalen Sequenzbereich ab der Aminosäure in Position 248 bis einschließlich Position 259 liegt, wobei Position 1 das Startmethionin aus Fig. 5 (SEQ ID NO: 1) ist und wobei die Mutation von Met zu Ile in Position 256 ausgeschlossen ist und daß die Serin-Acetyltransferase eine Inhibitorkonstante $K_i$ von 0,02 bis 2,3 mM in Gegenwart von 1 mM L-Serin und 0,1 mM Acetyl-CoA hat.

2. Serin-Acetyltransferase, die eine im Vergleich zum Wildtyp-Enzym reduzierte Sensitivität gegenüber dem Inhibitor L-Cystein aufweist und deren Proteinsequenz im Vergleich zur Wildtyp-Sequenz (SEQ ID NO:1) mindestens eine Mutation oder Deletion aufweist, **dadurch gekennzeichnet, daß** ihre Proteinsequenz die Aminosäureaustausche mindestens einer der in Tab. 1a oder 1b genannten cysE Mutanten umfaßt.

3. DNS-Sequenz, welche für eine Serin-Acetyltransferase gemäß einem der Ansprüche 1 oder 2 kodiert.

4. DNS-Sequenz, welche für eine Serin-Acetyltransferase kodiert, die eine im Vergleich zum Wildtyp-Enzym reduzierte Sensitivität gegenüber dem Inhibitor L-Cystein aufweist, **dadurch gekennzeichnet, daß** sie von bp 510 bis bp 1040 mindestens eine Mutation aufweisen,
wobei bp 1 die ersten Base aus Fig. 6 (SEQ ID NO: 2) ist, wobei die Mutation von Guanin nach Adenin, Thymidin oder Cytosin in Position 990 ausgeschlossen ist.

5. Mikroorganismen, **dadurch gekennzeichnet, daß** sie einen durch zumindest eine DNS-Sequenz gemäß Anspruch 3 oder 4 deregulierten Cysteinstoffwechsel besitzen.

6. Verfahren zur Herstellung von O-Acetylserin, L-Cystein oder von L-Cystein abgeleiteten Produkten, **dadurch gekennzeichnet, daß** Mikroorganismen gemäß Anspruch 5 in an sich bekannter Weise in einem Nährmedium kultiviert werden.

7. Verfahren gemäß Anspruch 6, **dadurch gekennzeichnet, daß** das Nährmedium eine ausreichende Menge an Schwefeldonoren enthält.

8. Verfahren gemäß Anspruch 7, **dadurch gekennzeichnet, daß** als Schwefeldonor Thiosulfat verwendet wird.

**Claims**

1. Serine acetyltransferase which exhibits a sensitivity to the inhibitor L-cysteine which is reduced in comparison to the wild-type enzyme and whose protein sequence exhibits at least one mutation or deletion when compared with the wild-type sequence, **characterized in that** the mutation is in the sequence region from the amino acid in position 97 up to and including the amino acid in position 273, or the deletion is in the carboxyterminal sequence region from the amino acid in position 248 up to and including position 259, with position 1 being the start methionine in Fig. 5 (SEQ ID NO: 1) and with the mutation of Met to Ile in position 256 being excluded, and **in that** the serine acetyltransferase has an inhibitor constant, $K_i$, of from 0.02 to 2.3 mM in the presence of 1 mM L-serine and 0.1 mM acetyl-CoA.

2. Serine acetyltransferase which exhibits a sensitivity to the inhibitor L-cysteine which is reduced in comparison to the wild-type enzyme and whose protein sequence exhibits at least one mutation or deletion when compared with the wild-type sequence (SEQ ID NO: 1), **characterized in that** its protein sequence contains the amino acid substitutions of at least one of the cysE mutants specified in Tab. 1a or 1b.

3. DNA sequence which encodes a serine acetyltransferase according to one of Claims 1 or 2.

4. DNA sequence which encodes the serine acetyltransferase which exhibits a sensitivity to the inhibitor L-cysteine which is reduced as compared with the wild-type enzyme, **characterized in that** it possesses at least one mutation from bp 510 to bp 1040, with bp 1 being the first base in Fig. 6 (SEQ ID NO: 2) and with the mutation of guanine to adenine, thymidine or cytosine in position 990 being excluded.

5. Microorganism **characterized in that** it possesses a cysteine metabolism which is deregulated by at least one DNA sequence according to Claim 3 or 4.

6. Process for preparing O-acetylserine, L-cysteine or products which are derived from L-cysteine, **characterized in that** the microorganisms are cultured according to Claim 5 in a nutrient medium in a manner known per se.

7. Process according to Claim 6, **characterized in that** the nutrient medium contains an adequate quantity of sulphur donors.

8. Process according to Claim 7, **characterized in that** thiosulphate is used as the sulphur donor.

**Revendications**

1. Sérine-acétyltransférase, qui présente, par rapport à l'enzyme de type sauvage, une sensibilité réduite par rapport à l'inhibiteur L-cystéine et dont la séquence protéinique par rapport à la séquence du type sauvage présente au moins une mutation ou délétion, **caractérisée en ce que** la mutation est située dans la zone de séquence depuis l'acide aminé en position 97 jusqu'à et y compris l'acide aminé en position 273 ou la délétion est située dans la zone de séquence à terminaison carboxy à partir de l'acide aminé en position 248 jusqu'à et y compris la position 259, la position 1 étant la méthionine de départ de la Fig. 5 (SEQ. ID NO : 1) et la mutation de Met en Ile en position 256 étant exclue et **en ce que** la sérine-acétyltransférase présente une constante d'inhibition $K_i$ de 0,02 à 2,3 mM en présence de L-sérine à 1 mM et d'acétyl-CoA à 0,1 mM.

**2.** Sérine-acétyltransférase, qui présente, par rapport à l'enzyme de type sauvage, une sensibilité réduite par rapport à l'inhibiteur L-cystéine et dont la séquence protéinique par rapport à la séquence du type sauvage (SEQ. ID NO : 1) présente au moins une mutation ou délétion, **caractérisée en ce que** sa séquence protéinique comprend les remplacements d'acide aminé d'au moins un des mutants cysE mentionnés dans le Tableau 1a ou 1b.

**3.** Séquence d'ADN qui code pour une sérine-acétyltransférase selon l'une quelconque des revendications 1 ou 2.

**4.** Séquence d'ADN qui code pour une sérine-acétyltransférase qui présente, par rapport à l'enzyme de type sauvage, une sensibilité réduite par rapport à l'inhibiteur L-cystéine, **caractérisée en ce qu'**elle présente au moins une mutation de bp 510 à bp 1040, bp 1 étant la première base de la Fig. 6 (SEQ. ID NO : 2), la mutation de guanidine en adénine, thymidine ou cytosine en position 990 étant exclue.

**5.** Microorganismes, **caractérisés en ce qu'**ils présentent un métabolisme de la cystéine dérégulé par au moins une séquence d'ADN selon la revendication 3 ou 4.

**6.** Procédé pour la production de O-acétylsérine, de L-cystéine ou de produits dérivés de la L-cystéine, **caractérisé en ce que** des microorganismes selon la revendication 5 sont cultivés de manière connue en soi dans un milieu nutritif.

**7.** Procédé selon la revendication 6, **caractérisé en ce que** le milieu nutritif contient une quantité suffisante de donneurs de soufre.

**8.** Procédé selon la revendication 7, **caractérisé en ce qu'**on utilise du thiosulfate comme donneur de soufre.

**Fig. 1**

Homoserin → (Succinyl-CoA, met A, ①) → O-Succinylhomoserin → (Cystein, Succinat, met B, ②) → Cystathionin → (NH₃, Pyruvat, met C, ③) → Homocystein → (CH₃THF, met E, met H, ④) → Methionin

① Homoserin-Succinyltransferase   ③ Cystathionin-β–Lyase

② Cystathionin-γ-Synthase   ④ Homocystein-Methylase

CH₃THF: N⁵-Methyltetrahydrofolat

EP 0 858 510 B1

## Fig. 2

COO⁻
|
CH₂
|
CH₂        ATP      ADP + Pi
|
H—C—NH₃⁺
|              L-Cystein
COO⁻           GSH I
                 ①

L-Glutamat

CO—NH—CH—COO⁻
|            |
CH₂          CH₂
|            |
CH₂          SH
|                      ATP    ADP + Pi
H—C—NH₃⁺
|
COO⁻           L-Glycin
                 GSH II
                  ②

L-γ-Glutamylcystein

                              COO⁻
                              |
                    CO—NH—CH₂
CO—NH—CH
|         |
CH₂       CH₂
|         |
CH₂       SH
|
H—C—NH₃⁺
|
COO⁻

Glutathion (GSH)

① γ-Glutamylcystein-Synthetase
② Glutathion-Synthetase

**Fig. 3**

Dethiobiotin → [Schwefel-Quelle (Cystein, GSH); bio B ①] → d-Biotin → [bio A ②] → Biotinyl-5-Adenylat

① Biotin Synthetase
② Biotin Holoenzym-Synthetase

EP 0 858 510 B1

EP 0 858 510 B1

## Fig. 4

Glucose → → 3-Phosphoglycerat $\xrightarrow[\text{ser A}\ \textcircled{1}]{\text{NAD+} \quad \text{NADH}}$ 3-Phospho-hydroxypyruvat $\xrightarrow[\text{ser C}\ \textcircled{2}]{\text{Transaminase}}$ 3-Phosphoserin →

$\xrightarrow[\text{ser B}\ \textcircled{3}]{\text{Phosphatase}}$ L-Serin $\xrightarrow[\text{cys E}\ \textcircled{4}]{\text{Acetyl-CoA} \quad \text{CoASH}}$ O-Acetylserin $\xrightarrow[\text{cysMK}\ \textcircled{5}]{S^{2-}}$ L-Cystein

① 3-Phosphoglycerat-Dehydrogenase

② 3-Phosphoserin-Aminotransferase

③ 3-Phosphoserin-Phosphatase

④ L-Serin-Acetyltransferase

⑤ O-Acetylserin-Sulfhydrylase

_Fig. 5_

```
  1  MSCEELEIVW NNIKAEARTL ADCEPMLASF YHATLLKHEN LGSALSYMLA

 51  NKLSSPIMPA IAIREVVEEA YAADPEMIAS AACDIQAVRT RDPAVDKYST

101  PLLYLKGFHA LQAYRIGHWL WNQGRRALAI FLQNQVSVTF QVDIHPAAKI

151  GRGIMLDHAT GIVVGETAVI ENDVSILQSV TLGGTGKSGG DRHPKIREGV

201  MIGAGAKILG NIEVGRGAKI GAGSVVLQPV PPHTTAAGVP ARIVGKPDSD

251  KPSMDMDQHF NGINHTFEYG DGI
```

# Fig. 6

```
   1  TCCGCGAACTGGCGCATCGCTTCGGCGTTGAAATGCCAATAACCGAGGAAATTTATCAAG

  61  TATTATATTGCGGAAAAAACGCGCGCGAGGCAGCATTGACTTTACTAGGTCGTGCACGCA

 121  AGGACGAGCGCAGCAGCCACTAACCCCAGGGAACCTTTGTTACCGCTATGACCCGGCCCG

 181  CGCAGAACGGGCCGGTCATTATCTCATCGTGTGGAGTAAGCAATGTCGTGTGAAGAACTG
   1                                                  MetSerCysGluGluLeu

 241  GAAATTGTCTGGAACAATATTAAAGCCGAAGCCAGAACGCTGGCGGACTGTGAGCCAATG
   7  GluIleValTrpAsnAsnIleLysAlaGluAlaArgThrLeuAlaAspCysGluProMet

 301  CTGGCCAGTTTTTACCACGCGACGCTACTCAAGCACGAAAACCTTGGCAGTGCACTGAGC
  27  LeuAlaSerPheTyrHisAlaThrLeuLeuLysHisGluAsnLeuGlySerAlaLeuSer

 361  TACATGCTGGCGAACAAGCTGTCATCGCCAATTATGCCTGCTATTGCTATCCGTGAAGTG
  47  TyrMetLeuAlaAsnLysLeuSerSerProIleMetProAlaIleAlaIleArgGluVal

 421  GTGGAAGAAGCCTACGCCGCTGACCCGGAAATGATCGCCTCTGCGGCCTGTGATATTCAG
  67  ValGluGluAlaTyrAlaAlaAspProGluMetIleAlaSerAlaAlaCysAspIleGln

 481  GCGGTGCGTACCCGCGACCCGGCAGTCGATAAATACTCAACCCCGTTGTTATACCTGAAG
  87  AlaValArgThrArgAspProAlaValAspLysTyrSerThrProLeuLeuTyrLeuLys

 541  GGTTTTCATGCCTTGCAGGCCTATCGCATCGGTCACTGGTTGTGGAATCAGGGGCGTCGC
 107  GlyPheHisAlaLeuGlnAlaTyrArgIleGlyHisTrpLeuTrpAsnGlnGlyArgArg

 601  GCACTGGCAATCTTTCTGCAAAACCAGGTTTCTGTGACGTTCCAGGTCGATATTCACCCG
 127  AlaLeuAlaIlePheLeuGlnAsnGlnValSerValThrPheGlnValAspIleHisPro

 661  GCAGCAAAAATTGGTCGCGGTATCATGCTTGACCACGCGACAGGCATCGTCGTTGGTGAA
 147  AlaAlaLysIleGlyArgGlyIleMetLeuAspHisAlaThrGlyIleValValGlyGlu

 721  ACGGCGGTGATTGAAAACGACGTATCGATTCTGCAATCTGTGACGCTTGGCGGTACGGGT
 167  ThrAlaValIleGluAsnAspValSerIleLeuGlnSerValThrLeuGlyGlyThrGly

 781  AAATCTGGTGGTGACCGTCACCCGAAAATTCGTGAAGGTGTGATGATTGGCGCGGGCGCG
 187  LysSerGlyGlyAspArgHisProLysIleArgGluGlyValMetIleGlyAlaGlyAla

 841  AAAATCCTCGGCAATATTGAAGTTGGGCGCGGCGCGAAGATTGGCGCAGGTTCCGTGGTG
 207  LysIleLeuGlyAsnIleGluValGlyArgGlyAlaLysIleGlyAlaGlySerValVal

 901  CTGCAACCGGTGCCGCCGCATACCACCGCCGCTGGCGTTCCGGCTCGTATTGTCGGTAAA
 227  LeuGlnProValProProHisThrThrAlaAlaGlyValProAlaArgIleValGlyLys

 961  CCAGACAGCGATAAGCCATCAATGGATATGGACCAGCATTTCAACGGTATTAACCATACA
 247  ProAspSerAspLysProSerMetAspMetAspGlnHisPheAsnGlyIleAsnHisThr

1021  TTTGAGTATGGGGATGGGATCTAATGTCCTGTGATCGTGCCGGATGCGATGTAATCATCT
 267  PheGluTyrGlyAspGlyIleEnd

1081  ATCCGGCCTACAGTAACTAATCTCTCAATACCGCTCCCGGATACCCCAACTGTCG-1135
```

**Fig. 1**

Restriktionskarte des Plasmids pP1

———— pUC18; ▬▬▬▬ chromosomale DNA; PL: Polylinker

Fig. 8

pPC43
4,1 kb

SacI
Polylinker
BamHI

cysE

StuI

ClaI

EcoRI
Polylinker
KpnI

bla

ScaI

Fig. 9


SEQUENZPROTOKOLL

(1) ALLGEMEINE ANGABEN:

(i) ANMELDER:

    (A) NAME: Consortium fuer elektrochemische Industrie, GmbH
    (B) STRASSE: Zielstattstr. 20
    (C) ORT: Muenchen
    (D) BUNDESLAND: Bayern
    (E) LAND: Germany
    (F) POSTLEITZAHL: 81379
    (G) TELEFON: 089/748440
    (H) TELEFAX: 089/74844350
    (I) TELEX: 5215553 cons d

(ii) BEZEICHNUNG DER ERFINDUNG: Verfahren zur Herstellung von O-Acetylserin, L-Cystein und L-Cystein-verwandten Produkten

(iii) ANZAHL DER SEQUENZEN: 24

(iv) COMPUTER-LESBARE FASSUNG:

    (A) DATENTRÄGER: Floppy disk
    (B) COMPUTER: IBM PC compatible
    (C) BETRIEBSSYSTEM: PC-DOS/MS-DOS
    (D) SOFTWARE: PatentIn Release #1.0, Version #1.30 (EPA)

(2) ANGABEN ZU SEQ ID NO: 1:

    (i) SEQUENZKENNZEICHEN:

        (A) LÄNGE: 273 Aminosäuren
        (B) ART: Aminosäure
        (C) STRANGFORM:
        (D) TOPOLOGIE: linear

    (ii) ART DES MOLEKÜLS: Protein

    (vi) URSPRÜNLICHE HERKUNFT:

        (A) ORGANISMUS: Escherichia coli
        (B) STAMM: W3110

    (xi) SEQUENZBESCHREIBUNG: SEQ ID NO: 1:

```
Met Ser Cys Glu Glu Leu Glu Ile Val Trp Asn Asn Ile Lys Ala Glu
1               5               10              15

Ala Arg Thr Leu Ala Asp Cys Glu Pro Met Leu Ala Ser Phe Tyr His
            20              25              30

Ala Thr Leu Leu Lys His Glu Asn Leu Gly Ser Ala Leu Ser Tyr Met
        35              40              45

Leu Ala Asn Lys Leu Ser Ser Pro Ile Met Pro Ala Ile Ala Ile Arg
        50              55              60

Glu Val Val Glu Glu Ala Tyr Ala Ala Asp Pro Glu Met Ile Ala Ser
65              70              75              80
```

```
Ala Ala Cys Asp Ile Gln Ala Val Arg Thr Arg Asp Pro Ala Val Asp
                85                      90                      95

Lys Tyr Ser Thr Pro Leu Leu Tyr Leu Lys Gly Phe His Ala Leu Gln
            100                 105                 110

Ala Tyr Arg Ile Gly His Trp Leu Trp Asn Gln Gly Arg Arg Ala Leu
            115                 120                 125

Ala Ile Phe Leu Gln Asn Gln Val Ser Val Thr Phe Gln Val Asp Ile
        130                 135                 140

His Pro Ala Ala Lys Ile Gly Arg Gly Ile Met Leu Asp His Ala Thr
145                 150                 155                 160

Gly Ile Val Val Gly Glu Thr Ala Val Ile Glu Asn Asp Val Ser Ile
                165                 170                 175

Leu Gln Ser Val Thr Leu Gly Gly Thr Gly Lys Ser Gly Gly Asp Arg
            180                 185                 190

His Pro Lys Ile Arg Glu Gly Val Met Ile Gly Ala Gly Ala Lys Ile
            195                 200                 205

Leu Gly Asn Ile Glu Val Gly Arg Gly Ala Lys Ile Gly Ala Gly Ser
        210                 215                 220

Val Val Leu Gln Pro Val Pro Pro His Thr Thr Ala Ala Gly Val Pro
225                 230                 235                 240

Ala Arg Ile Val Gly Lys Pro Asp Ser Asp Lys Pro Ser Met Asp Met
                245                 250                 255

Asp Gln His Phe Asn Gly Ile Asn His Thr Phe Glu Tyr Gly Asp Gly
            260                 265                 270

Ile
```

(2) ANGABEN ZU SEQ ID NO: 2:

    (i) SEQUENZKENNZEICHEN:

        (A) LÄNGE: 1135 Basenpaare
        (B) ART: Nucleotid
        (C) STRANGFORM: Doppelstrang
        (D) TOPOLOGIE: linear

    (ii) ART DES MOLEKÜLS: Genom-DNA

    (vi) URSPRÜNLICEE HERKUNFT:

        (A) ORGANISMUS: Escherichia coli
        (B) STAMM: W3110

    (vii) UNMITTELBARE HERKUNFT:

        (B) CLON(E): pPC43

(xi) SEQUENZBESCHREIBUNG: SEQ ID NO: 2:

```
TCCGCGAACT GGCGCATCGC TTCGGCGTTG AAATGCCAAT AACCGAGGAA ATTTATCAAG      60

TATTATATTG CGGAAAAAAC GCGCGCGAGG CAGCATTGAC TTTACTAGGT CGTGCACGCA     120

AGGACGAGCG CAGCAGCCAC TAACCCCAGG GAACCTTTGT TACCGCTATG ACCCGGCCCG     180

CGCAGAACGG GCCGGTCATT ATCTCATCGT GTGGAGTAAG CAATGTCGTG TGAAGAACTG     240

GAAATTGTCT GGAACAATAT TAAAGCCGAA GCCAGAACGC TGGCGGACTG TGAGCCAATG     300

CTGGCCAGTT TTTACCACGC GACGCTACTC AAGCACGAAA ACCTTGGCAG TGCACTGAGC     360

TACATGCTGG CGAACAAGCT GTCATCGCCA ATTATGCCTG CTATTGCTAT CCGTGAAGTG     420

GTGGAAGAAG CCTACGCCGC TGACCCGGAA ATGATCGCCT CTGCGGCCTG TGATATTCAG     480

GCGGTGCGTA CCCGCGACCC GGCAGTCGAT AAATACTCAA CCCCGTTGTT ATACCTGAAG     540

GGTTTTCATG CCTTGCAGGC CTATCGCATC GGTCACTGGT TGTGGAATCA GGGGCGTCGC     600

GCACTGGCAA TCTTTCTGCA AAACCAGGTT TCTGTGACGT TCCAGGTCGA TATTCACCCG     660

GCAGCAAAAA TTGGTCGCGG TATCATGCTT GACCACGCGA CAGGCATCGT CGTTGGTGAA     720

ACGGCGGTGA TTGAAAACGA CGTATCGATT CTGCAATCTG TGACGCTTGG CGGTACGGGT     780

AAATCTGGTG GTGACCGTCA CCCGAAAATT CGTGAAGGTG TGATGATTGG CGCGGGCGCG     840

AAAATCCTCG GCAATATTGA AGTTGGGCGC GGCGCGAAGA TTGGCGCAGG TTCCGTGGTG     900

CTGCAACCGG TGCCGCCGCA TACCACCGCC GCTGGCGTTC CGGCTCGTAT TGTCGGTAAA     960

CCAGACAGCG ATAAGCCATC AATGGATATG GACCAGCATT TCAACGGTAT TAACCATACA    1020

TTTGAGTATG GGGATGGGAT CTAATGTCCT GTGATCGTGC CGGATGCGAT GTAATCATCT    1080

ATCCGGCCTA CAGTAACTAA TCTCTCAATA CCGCTCCCGG ATACCCCAAC TGTCG         1135
```

(2) ANGABEN ZU SEQ ID NO: 3:

  (i) SEQUENZKENNZEICHEN:

    (A) LÄNGE: 42 Basenpaare
    (B) ART: Nucleotid
    (C) STRANGFORM: Einzelstrang
    (D) TOPOLOGIE: linear

  (ii) ART DES MOLEKÜLS: Sonstige Nucleinsäure

    (A) BESCHREIBUNG: /desc = "oligonucleotide"

  (vii) UNMITTELBARE HERKUNFT:

    (A) BIBLIOTHEK: synthetic
    (B) CLON(E): cysE-fw1

(xi) SEQUENZBESCHREIBUNG: SEQ ID NO: 3:

```
GCCTGGATCC TGCAGTCGAC CTGGCGCATC GCTTCGGCGT TG                    42
```

(2) ANGABEN ZU SEQ ID NO: 4:

(i) SEQUENZKENNZEICHEN:

(A) LÄNGE: 41 Basenpaare
(B) ART: Nucleotid
(C) STRANGFORM: Einzelstrang
(D) TOPOLOGIE: linear

(ii) ART DES MOLEKÜLS: Sonstige Nucleinsäure

(A) BESCHREIBUNG: /desc = "oligonucleotide"

(vii) UNMITTELBARE HERKUNFT:

(A) BIBLIOTHEK: synthetic
(B) CLOM(E): cysE-rev1

(xi) SEQUENZBESCHREIBUNG: SEQ ID NO: 4:

```
GTAGGAGCTC TGCAGAATTC GGGTATCCGG GAGCGGTATT G                     41
```

(2) ANGABEN ZU SEQ ID NO: 5:

(i) SEQUENZKENNZEICHEN:

(A) LÄNGE: 18 Basenpaare
(B) ART: Nucleotid
(C) STRANGFORM: Einzelstrang
(D) TOEOLOGIE: linear

(ii) ART DES MOLEKÜLS: Sonstige Nucleinsäure

(A) BESCHREIBUNG: /desc = "oligonucleotide"

(vii) UNMITTELBARE HERKUNFT:

(A) BIBLIOTHEK: synthetic
(B) CLON(E): cysE-Mut-1

(xi) SEQUENZBESCHREIBUNG: SEQ ID NO: 5:

```
GCCGCTAGCG TTCCGGCT                                               18
```

(2) ANGABEN ZU SEQ ID NO: 6:

(i) SEQUENZKENNZEICHEN:

(A) LÄNGE: 21 Basenpaare

(B) ART: Nucleotid
(C) STRANGFORM: Einzelstrang
(D) TOPOLOGIE: linear

(ii) ART DES MOLEKÜLS: Sonstige Nucleinsäure

(A) BESCHREIBUNG: /desc = "oligonucleotide"

(vii) UNMITTELBARE HERKUNFT:

(A) BIBLIOTHEK: synthetic
(B) CLON(E): cysE-Mut-3

(xi) SEQUENZBESCHREIBUNG: SEQ ID NO: 6:

CCGCCGCATA CCACCGCCGT T                                      21

(2) ANGABEN ZU SEQ ID NO: 7:

(i) SEQUENZKENNZEICHEN:

(A) LÄNGE: 24 Basenpaare
(B) ART: Nucleotid
(C) STRANGFORM: Einzelstrang
(D) TOPOLOGIE: linear

(ii) ART DES MOLEKÜLS: Sonstige Nucleinsäure

(A) BESCHREIBUNG: /desc = "oligonucleotide"

(vii) UNMITTELBARE HERKUNFT:

(A) BIBLIOTHEK: synthetic
(B) CLON(E): cysE-Mut-6

(xi) SEQUENZBESCHREIBUNG: SEQ ID NO: 7:

CCATCAATGG ATATAGACCA GCAT                                   24

(2) ANGABEN ZU SEQ ID NO: 8:

(i) SEQUENZKENNZEICHEN:

(A) LANGE: 24 Basenpaare
(B) ART: Nucleotid
(C) STRANGFORM: Einzelstrang
(D) TOPOLOGIE: linear

(ii) ART DES MOLEKÜLS: Sonstige Nucleinsäure

(A) BESCHREIBUNG: /desc = "oligonucleotide"

(vii) UNMITTELBARE HERKUNFT:

(A) BIBLIOTHEK: synthetic

(B) CLON(E): cysE-Mut-10

(xi) SEQUENZBESCHREIBUNG: SEQ ID NO: 8:

GTCGTTGGTG AAGCGGCGGT GATT                                                                24

(2) ANGABEN ZU SEQ ID NO: 9:

(i) SEQUENZKENNZEICHEN:

(A) LÄNGE: 38 Basenpaare
(B) ART: Nucleotid
(C) STRANGFORM: Einzelstrang
(D) TOPOLOGIE: linear

(ii) ART DES MOLEKÜLS: Sonstige Nucleinsäure

(A) BESCHREIBUNG: /desc = "oligonucleotide"

(vii) UNMITTELBARE HERKUNFT:

(A) BIBLIOTHEK: synthetic
(B) CLON(E): cysE-LHfw1

(xi) SEQUENZBESCHREIBUNG: SEQ ID NO: 9:

TGGACCAGAG CTCTGGCTGG CGCATCGCTT CGGCGTTG                                                38

(2) ANGABEN ZU SEQ ID NO: 10:

(i) SEQUENZKENNZEICHEN:

(A) LANGE: 46 Basenpaare
(B) ART: Nucleotid
(C) STRANGFORM: Einzelstrang
(D) TOPOLOGIE: linear

(ii) ART DES MOLEKÜLS: Sonstige Nucleinsäure

(A) BESCHREIBUNG: /desc = "oligonucleotide"

(vii) UNMITTELBARE HERKUNFT:

(A) BIBLIOTHEK: synthetic
(B) CLON(E): cysE-Del270

(xi) SEQUENZBESCHREIBUNG: SEQ ID NO: 10:

CTCGATGCAT TACGTATTAC CCATACTCAA ATCTATGGTT AATACC                                       46

(2) ANGABEN ZU SEQ ID NO: 11:

(i) SEQUENZKENNZEICHEN:

(A) LÄNGE: 46 Basenpaare
(B) ART: Nucleotid
(C) STRANGFORM: Einzelstrang
(D) TOPOLOGIE: linear

(ii) ART DES MOLEKÜLS: Sonstige Nucleinsäure

(A) BESCHREIBUNG: /desc = "oligonucleotide"

(vii) UNMITTELBARE HERKUNFT:

(A) BIBLIOTHEK: synthetic
(B) CLON(E): cysE-De1268

(xi) SEQUENZBESCHREIBUNG: SEQ ID NO: 11:

```
CTCGATGCAT TACGTATTAC TCAAATGTAT GGTTAATACC GTTGAA          46
```

(2) ANGABEN ZU SEQ ID NO: 12:

(i) SEQUENZKENNZEICHEN:

(A) LÄNGE: 46 Basenpaare
(B) ART: Nucleotid
(C) STRANGFORM: Einzelstrang
(D) TOPOLOGIE: linear

(ii) ART DES MOLEKÜLS: Sonstige Nucleinsäure

(A) BESCHREIBUNG: /desc = "oligonucleotide"

(vii) UNMITTELBARE HERKUNFT:

(A) BIBLIOTHEK: synthetic
(B) CLON(E): cysE-Del263

(xi) SEQUENZBESCHREIBUNG: SEQ ID NO: 12:

```
CTCGATGCAT TACGTATTAA ATACCGTTGA AATGCTGGTC CATATC          46
```

(2) ANGABEN ZU SEQ ID NO: 13:

(i) SEQUENZKENNZEICHEN:

(A) LÄNGE: 46 Basenpaare
(B) ART: Nucleotid
(C) STRANGFORM: Einzelstrang
(D) TOPOLOGIE: linear

(ii) ART DES MOLEKÜLS: Sonstige Nucleinsäure

(A) BESCHREIBUNG: /desc = "oligonucleotide"

(vii) UNMITTELBARE HERKUNFT:

(A) BIBLIOTHEK: synthetic

(B) CLOK(E): cysE-Del259

(xi) SEQUENZBESCHREIBUNG: SEQ ID NO: 13:

CTCGATGCAT TACGTATTAA TGCTGGTCCA TATCCATTGA TGGCTT                46

(2) ANGABEN ZU SEQ ID NO: 14:

(i) SEQUENZKENNZEICHEN:

(A) LÄNGE: 46 Basenpaare
(B) ART: Nucleotid
(C) STRANGFORM: Einzelstrang
(D) TOPOLOGIE: linear

(ii) ART DES MOLEKÜLS: Sonstige Nucleinsäure

(A) BESCHREIBUNG: /desc = "oligonucleotide"

(vii) UNMITTELBARE HERKUNFT:

(A) BIBLIOTHEK: synthetic
(B) CLON(E): cysE-Del258

(xi) SEQUENZBESCHREIBUNG: SEQ ID NO: 14:

CTCGATGCAT TACGTATTAC TGGTCCATAT CCATTGATGG CTTATC                46

(2) ANGABEN ZU SEQ ID NO: 15:

(i) SEQUENZYENNZEICHEN:

(A) LÄNGE: 47 Basenpaare
(B) ART: Nucleotid
(C) STRANGFORM: Einzelstrang
(D) TOPOLOGIE: linear

(ii) ART DES MOLEKÜLS: Sonstige Nucleinsäure

(A) BESCHREIBUNG: /desc = "oligonucleotide"

(vii) UNMITTELBARE HERKUNFT:

(A) BIBLIOTHEK: synthetic
(B) CLON(E): cysE-Del257

(xi) SEQUENZBESCHREIBUNG: SEQ ID NO: 15:

CTCGATGCAT TACGTATTAG TCCATATCCA TTGATGGCTT ATCGCTG                47

(2) ANGABEN ZU SEQ ID NO: 16:

(i) SEQUENZKENNZEICHEN:

(A) LANGE: 46 Basenpaare
(B) ART: Nucleotid
(C) STRANGFORM: Einzelstrang
(D) TOPOLOGIE: linear

(ii) ART DES MOLEKÜLS: Sonstige Nucleinsäure

(A) BESCHREIBUNG: /desc = "oligonucleotide"

(vii) UNMITTELBARE HERKUNFT:

(A) BIBLIOTHEK: synthetic
(B) CLON(E): cysE-Del256

(xi) SEQUENZBESCHREIBUNG: SEQ ID NO: 16:


CTCGATGCAT TACGTATTAC ATATCCATTG ATGGCTTATC GCTGTC                     46


(2) ANGABEN ZU SEQ ID NO: 17:

(i) SEQUENZKENNZEICHEN:

(A) LÄNGE: 46 Basenpaare
(B) ART: Nucleotid
(C) STRANGFORM: Einzelstrang
(D) TOPOLOGIE: linear

(ii) ART DES MOLEKÜLS: Sonstige Nucleinsäure

(A) BESCHREIBUNG: /desc = "oligonucleotide"

(vii) UNMITTELBARE HERKUNFT:

(A) BIBLIOTHEK: synthetic
(B) CLON(E): cysE-Del255

(xi) SEQUENZBESCHREIBUNG: SEQ ID NO: 17:


CTCGATGCAT TACGTATTAA TCCATTGATG GCTTATCGCT GTCTGG                     46


(2) ANGABEN ZU SEQ ID NO: 18:

(i) SEQUENZKENNZEICHEN:

(A) LÄNGE: 46 Basenpaare
(B) ART: Nucleotid
(C) STRANGFORM: Einzelstrang
(D) TOPOLOGIE: linear

(ii) ART DES MOLEKÜLS: Sonstige Nucleinsäure

(A) BESCHREIBUNG: /desc = "oligonucleotide"

(vii) UNMITTELBARE HERKUNFT:

(A) BIBLIOTHEK: synthetic
(B) CLON(E): cysE-Del250

(xi) SEQUENZBESCHREIBUNG: SEQ ID NO: 18:

```
CTCGATGCAT TACGTATTAA TCGCTGTCTG GTTTACCGAC AATACG          46
```

(2) ANGABEN ZU SEQ ID NO: 19:

(i) SEQUENZKENNZEICHEN:

(A) LÄNGE: 46 Basenpaare
(B) ART: Nucleotid
(C) STRANGFORM; Einzelstrang
(D) TOPOLOGIE: linear

(ii) ART DES MOLEKÜLS: Sonstige Nucleinsäure

(A) BESCHREIBUNG: /desc = "oligonucleotide"

(vii) UNMITTELBARE HERKUNFT:

(A) BIBLIOTHEK: synthetic
(B) CLON(E): cysE-Del249

(xi) SEQUENZBESCHREIBUNG: SEQ ID NO: 19:

```
CTCGATGCAT TACGTATTAG CTGTCTGGTT TACCGACAAT ACGAGC          46
```

(2) ANGABEN ZU SEQ ID NO: 20:

(i) SEQUENZKENNZEICHEN:

(A) LÄNGE: 46 Basenpaare
(B) ART: Nucleotid
(C) STRANGFORM: Einzelstrang
(D) TOPOLOGIE: linear

(ii) ART DES MOLEKÜLS: Sonstige Nucleinsäure

(A) BESCHREIBUNG: /desc = "oligonucleotide"

(vii) UNMITTELBARE HERKUNFT:

(A) BIBLIOTHEK: synthetic
(B) CLON(E): cysE-Del248

(xi) SEQUENZBESCHREIBUNG: SEQ ID NO: 20:

```
CTCGATGCAT TACGTATTAG TCTGGTTTAC CGACAATACG AGCCGG          46
```

(2) ANGABEN ZU SEQ ID NO: 21:

(i) SEQUENZKENNZEICHEN:

(A) LÄNGE: 46 Basenpaare
(B) ART: Nucleotid
(C) STRANGFORM: Einzelstrang
(D) TOPOLOGIE: linear

(ii) ART DES MOLEKÜLS: Sonstige Nucleinsäure

(A) BESCHREIBUNG: /desc = "oligonucleotide"

(vii) UNMITTELBARE HERKUNFT:

(A) BIBLIOTHEK: synthetic
(B) CLON(E): cysE-Del245

(xi) SEQUENZBESCHREIBUNG: SEQ ID NO: 21:

```
CTCGATGCAT TACGTATTAA CCGACAATAC GAGCCGGAAC GCCAGC                    46
```

(2) ANGABEN ZU SEQ ID NO: 22:

(i) SEQUENZKENNZEICHEN:

(A) LANGE: 46 Basenpaare
(B) ART: Nucleotid
(C) STRANGFORM: Einzelstrang
(D) TOPOLOGIE: linear

(ii) ART DES MOLEKÜLS: Sonstige Nucleinsäure

(A) BESCHREIBUNG: /desc = "oligonucleotide"

(vii) UNMITTELBARE HERKUNFT:

(A) BIBLIOTHEK: synthetic
(B) CLON(E): cysE-Del239

(xi) SEQUENZBESCHREIBUNG: SEQ ID NO: 22:

```
CTCGATGCAT TACGTATTAA ACGCCAGCGG CGGTGGTATC CGGCGG                    46
```

(2) ANGABEN ZU SEQ ID NO: 23:

(i) SEQUENZKENNZEICHEN:

(A) LÄNGE: 46 Basenpaare
(B) ART: Nucleotid
(C) STRANGFORM: Einzelstrang
(D) TOPOLOGIE: linear

(ii) ART DES MOLEKÜLS: Sonstige Nucleinsäure

(A) BESCHREIBUNG: /desc = "oligonucleotide"

(vii) UNMITTELBARE HERKUNFT:

(A) BIBLIOTHEK: synthetic

        (B) CLON(E): cysE-Del227

    (xi) SEQUENZBESCHREIBUNG: SEQ ID NO: 23:


CTCGATGCAT TACGTATTAC AGCACCACGG AACCTGCGCC AATCTT                          46


 (2) ANGABEN ZU SEQ ID NO: 24:

    (i) SEQUENZKENNZEICHEN:

        (A) LÄNGE: 38 Basenpaare
        (B) ART: Nucleotid
        (C) STRANGFORM: Einzelstrang
        (D) TOPOLOGIE: linear

    (ii) ART DES MOLEKÜLS: Sonstige Nucleinsäure

        (A) BESCHREIBUNG: /desc = "oligonucleotide"

    (vii) UNMITTELBARE HERKUNFT:

        (A) BIBLIOTHEK: synthetic
        (B) CLON(E): cysE-LHrevI

    (xi) SEQUENZBESCHREIBUNG: SEQ ID NO: 24:


CTCGATGCAT TACGTAGGGG TATCCGGGAG CGGTATTG                          38